(19)

**Europäisches Patentamt**
**European Patent Office**
**Office européen des brevets**

(11) **EP 4 363 548 B1**

(12) **EUROPEAN PATENT SPECIFICATION**

(45) Date of publication and mention
of the grant of the patent:
**17.06.2026   Bulletin 2026/25**

(21) Application number: **22741742.5**

(22) Date of filing: **30.06.2022**

(51) International Patent Classification (IPC):
*C12M 1/12* (2006.01)      *C12M 1/42* (2006.01)
*C12P 7/04* (2006.01)      *C12P 7/26* (2006.01)
*C12P 7/62* (2022.01)      *C12P 17/12* (2006.01)
*C12P 19/02* (2006.01)

(52) Cooperative Patent Classification (CPC):
**C12P 7/04; C12M 25/16; C12M 35/06; C12P 7/26;**
**C12P 7/62; C12P 17/12; C12P 19/02;** Y02E 50/10

(86) International application number:
**PCT/EP2022/068190**

(87) International publication number:
**WO 2023/275321 (05.01.2023 Gazette 2023/01)**

(54) **NANOPARTICLES FOR THE CONTROL OF ONE-POT MULTI-ENZYMATIC REACTIONS**

NANOTEILCHEN ZUR STEUERUNG VON MULTIENZYMATISCHEN REAKTIONEN IN EINEM GEFÄSS

NANOPARTICULES POUR LE CONTRÔLE DE RÉACTIONS MULTI-ENZYMATIQUES DANS UN RECIPIENT

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority:  **01.07.2021   EP 21382585**

(43) Date of publication of application:
**08.05.2024   Bulletin 2024/19**

(73) Proprietors:
• **Consejo Superior De Investigaciones Científicas**
  **28006 Madrid (ES)**
• **CIC biomaGUNE**
  **20014 Donostia/San Sebastián (Gipuzkoa) (ES)**
• **Graz University Of Technology**
  **8010 Graz (AT)**
• **Forschungszentrum Jülich GmbH**
  **52425 Jülich (DE)**
• **Universita' Degli Studi Dell'Insubria**
  **21100 Varese (IT)**

(72) Inventors:
• **GARCÍA OVEJERO, Jesus**
  **28006 Madrid (ES)**

• **ARMENIA, Ilaria**
  **28006 Madrid (ES)**
• **VEINTEMILLAS VERDAGUER, Sabino**
  **28006 Madrid (ES)**
• **MARTÍNEZ DE LA FUENTE, Jesús**
  **28006 Madrid (ES)**
• **MORALES HERRERO, María del Puerto**
  **28006 Madrid (ES)**
• **GRAZÚ BONAVIA, María Valeria**
  **28006 Madrid (ES)**
• **GUISÁN SEIJAS, José Manuel**
  **28006 Madrid (ES)**
• **LÓPEZ GALLEGO, Fernando**
  **20014 Donostia/San Sebastián (Gipuzkoa) (ES)**
• **NIDETZKY, Bernd**
  **8010 Graz (AT)**
• **ROTHER, Dörte**
  **52425 Jülich (DE)**
• **CASSINELLI, Nicolas**
  **50197 Zaragoza (ES)**
• **BERNARDINI, Giovanni Battista**
  **21100 Varese (IT)**

**(Cont. next page)**

(74) Representative: **Hoffmann Eitle**
**Hoffmann Eitle S.L.U.**
**Paseo de la Castellana 140, 3a planta**
**Edificio LIMA**
**28046 Madrid (ES)**

(56) References cited:

- **GOLOVIN YURI I ET AL: "Towards nanomedicines of the future: Remote magneto-mechanical actuation of nanomedicines by alternating magnetic fields", JOURNAL OF CONTROLLED RELEASE, ELSEVIER, AMSTERDAM, NL, vol. 219, 25 September 2015 (2015-09-25), pages 43 - 60, XP029303670, ISSN: 0168-3659, DOI: 10.1016/J.JCONREL.2015.09.038**
- **DEL ARCO JON ET AL: "Magnetic micro-macro biocatalysts applied to industrial bioprocesses", BIORESOURCE TECHNOLOGY, ELSEVIER, AMSTERDAM, NL, vol. 322, 16 December 2020 (2020-12-16), XP086434469, ISSN: 0960-8524, [retrieved on 20201216], DOI: 10.1016/ J.BIORTECH.2020.124547**
- **YOLANDA MOLDES-DIZ ET AL: "A novel enzyme catalysis reactor based on superparamagnetic nanoparticles for biotechnological applications", JOURNAL OF ENVIRONMENTAL CHEMICAL ENGINEERING, vol. 6, no. 5, 1 October 2018 (2018-10-01), NL, pages 5950 - 5960, XP055575817, ISSN: 2213-3437, DOI: 10.1016/ j.jece.2018.09.014**
- **GOLOVIN YURI I ET AL: "Theranostic multimodal potential of magnetic nanoparticles actuated by non-heating low frequency magnetic field in the new-generation nanomedicine", JOURNAL OF NANOPARTICLE RESEARCH, SPRINGER NETHERLANDS, DORDRECHT, vol. 19, no. 2, 11 February 2017 (2017-02-11), pages 1 - 47, XP036156189, ISSN: 1388-0764, [retrieved on 20170211], DOI: 10.1007/S11051-017-3746-5**

**Description**

**TECHNICAL FIELD OF THE INVENTION**

The present invention relates to magnetic nanoparticles for the control of enzymatic reactions. Particularly, it refers to a process to carry out two or more simultaneous or sequential enzymatic reactions on the surface of magnetic nanoparticles in a one-pot system.

**BACKGROUND OF THE INVENTION**

Enzymes have higher selectivity, specificity and efficiency than chemical catalysts. Due to their properties and their green chemistry, biocatalysts are widely used in food, textile and pharmaceutical industry. Enzymatic and multi-enzymatic reactions have also become an important tool in biotechnology industry for the production of biopolymers, drugs or biofuels. An important limitation for the implementation of multi-enzymatic reactions in the industry is the optimization of enzymes performance, the different operational temperatures of the enzymes involved in the processes and the reduction of negative interactions among them. The multi-enzymatic reactions are usually performed in a multistep reactor or by operating at a compromised temperature. However, these approaches reduce the efficiency of the systems and result critical when thermolabile enzymes, substrates, co- factors or products are involved

DEL ARCO JON ET AL: "Magnetic micro-macro biocatalysts applied to industrial bioprocesses",BIORESOURCE TECHNOLOGY, ELSEVIER, AMSTERDAM, NL, vol. 322, 16 December 2020 (2020-12-16)discloses to recover and re-utilize immobilized enzymes and that magnetic properties of the support can be further exploited for the regulation of enzyme activity.

A novel strategy to overcome this limitation is the use of magnetic nanoparticles (MNPs) as local nanoheaters of the enzymes localized at the nanoparticle surface. In this context, MNPs can be used for the conjugation of enzymes thanks to their high specific surface area (o surface area per gram), high surface/volume ratio, low mass transfer limitation and their unique magnetic properties. Indeed, MNPs can be manipulated by an external alternating magnetic field (AMF) and, as a consequence of Neel and Brown relaxation, they can produce heat, a phenomenon known as magnetic hyperthermia. When an AMF is applied to a colloid of MNPs, the energy of the field is dissipated on the nanoparticle surface as heat. However, the use of magnetic heating to gain control over enzyme activity is nowadays an important limitation for the use of these systems since the effect of the heat generated by high frequencies of AMF on enzymes directly attached to MNPs has been though scarcely studied. Thus, a fine tuning of such magnetic activation is required among the suitable MNPs.

The amount of heat dissipated depends on the nanoparticle composition, size, shape and aggregation state, but also on the AMF conditions (frequency and field). Thus, the temperature induced on the surface of the MNPs needs to be carefully optimized to match the optimal operational working temperature ($T_{OPT}$) of the enzyme attached to the MNPS. This becomes particularly difficult in one-pot multi-enzymatic processes, when different enzymes attached to different MNPs want to be used in a single space, since each enzyme would need different optimal conditions to work at their maximum efficiency. Furthermore, the conditions may vary depending on whether the simultaneous or sequential activation of the different enzymes in the one-pot system is desired.

Thus, a panel of different MNPs conjugated with different enzymes to be used in a one-pot system has not been achieved so far since a fine control of the local temperature at the enzyme position is necessary together with a in deep expertise in MNPs used as nanoheaters and enzyme supporters and their behaviour under different AMF conditions.

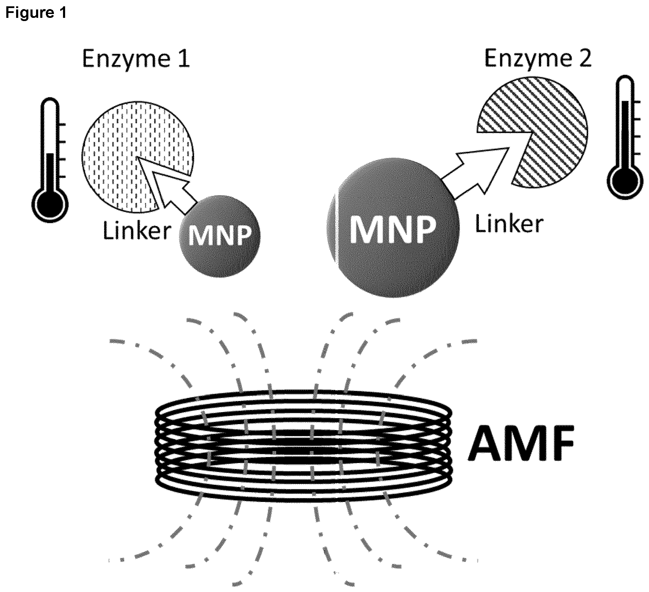

The present invention solves the above problem and it proposes a one-pot multienzymatic system of MNPs that allows the control of different local temperatures under the specific AMF conditions causing the simultaneous activation of the enzymes, or under sequential AMF conditions, causing the sequential activation of the enzymes (Figure 1). It is thus shown for the first time the feasibility of combining different MNPs with different enzymes to achieve a simultaneous or sequential enzymatic control of multienzymatic bioprocesses of industrial interest.

**DESCRIPTION OF THE FIGURES**

Figure 1. Schematic representation of a 2 magnetic nanoparticle (MNP)-enzyme systems thermally activated with alternating magnetic fields (AMF) for the simultaneous or sequential regulation of multi-enzymatic processes.

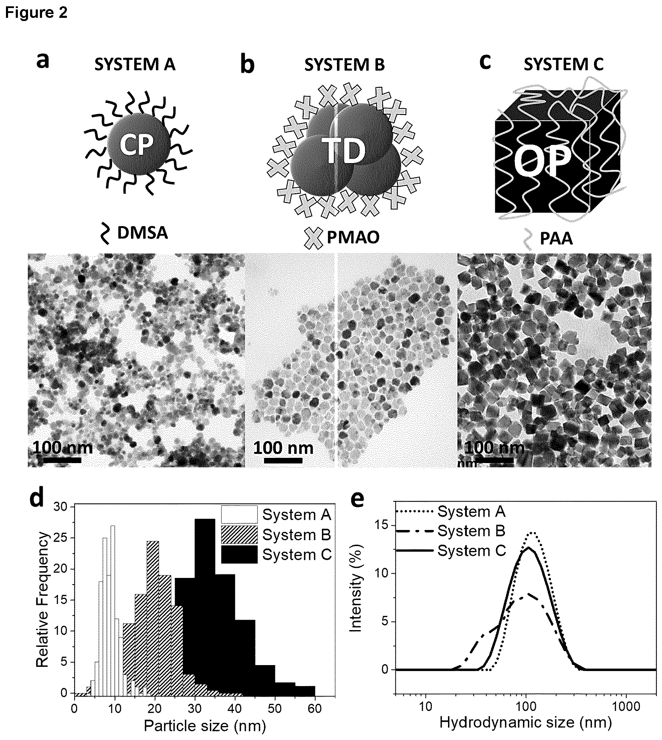

Figure 2. The three systems of magnetic nanoparticles (MNPs) A, B and C were produced by coprecipitation (CP), thermal decomposition (TD) and oxidative precipitation (OP), respectively. Scheme of geometry and coating of the MNPs and Transmission Electron Microscopy (TEM) pictures of a) System A, b) System B and c) System C. d) TEM size distributions. **e)** Dynamic Light Scattering (DLS) profiles of the hydrodynamic size distributions for the three systems.

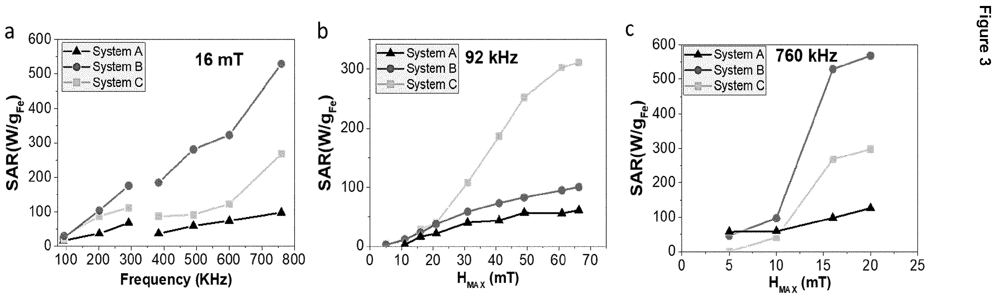

**Figure 3.** Magnetic heating properties of some of the three systems of MNPs at **a)** fixed $H_{MAX}$ =16 mT at different frequencies; **b)** fixed low frequency (92 kHz) at different $H_{max}$; **c)** fixed high frequency (760 kHz) at different $H_{max}$.

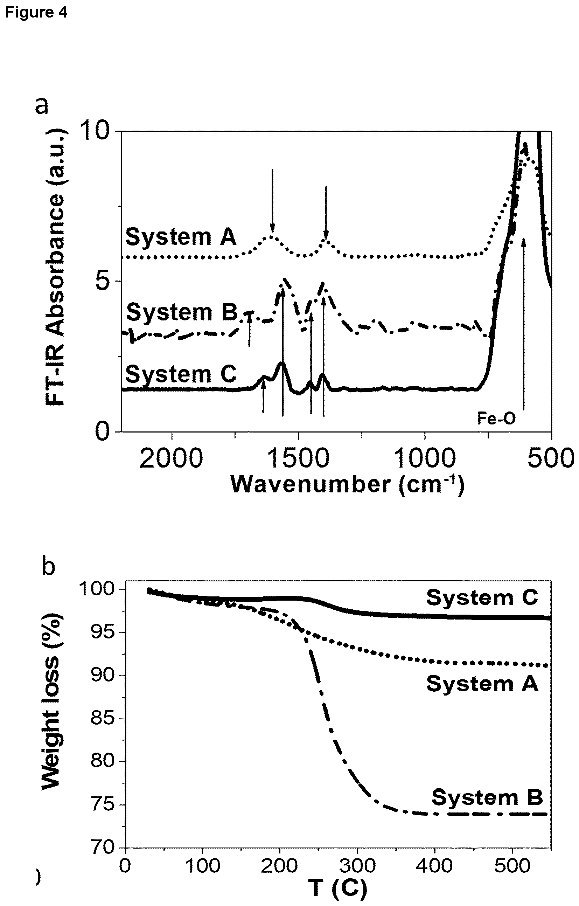

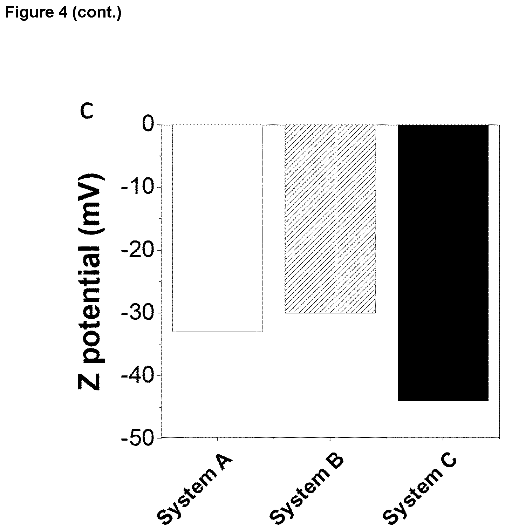

**Figure 4.** The surface of MNPs was decorated with carboxylic functional groups using organic coatings of DMSA, PMAO and PAA. **a)** Thermogravimetric (TG) curves, **b)** Fourier transformed Infrared (FT-IR) absorbance spectra and **c)** Z-potential values for systems A, B and C.

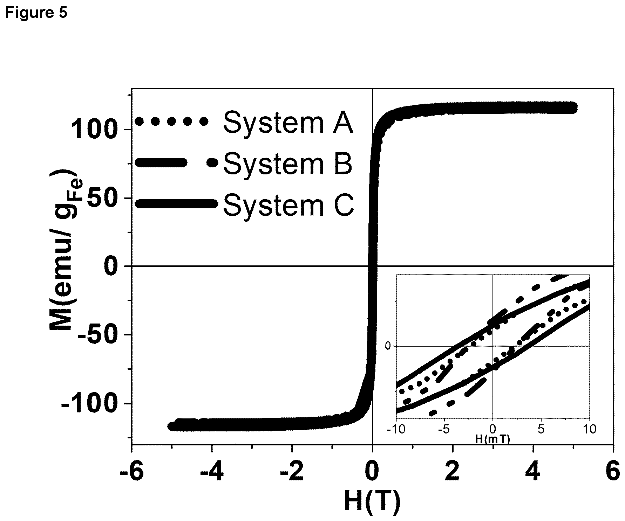

**Figure 5.** Magnetization-field hysteresis loops of the three systems of MNPs in quasistatic condition. The inset shows the central part of the graph.

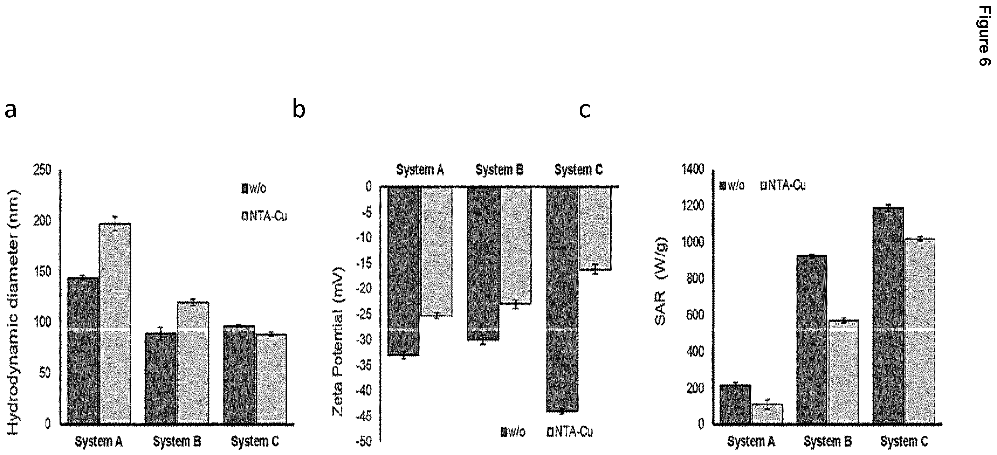

**Figure 6.** Physicochemical characterization of **A)** DLS, **B)** Z potential and **C)** SAR of the MNPs before and after functionalizing with NTA-Cu$^{+2}$. Left column of each group: non-functionalized MNPs; Right column: MNPs functionalized with NTA-Cu$^{+2}$.

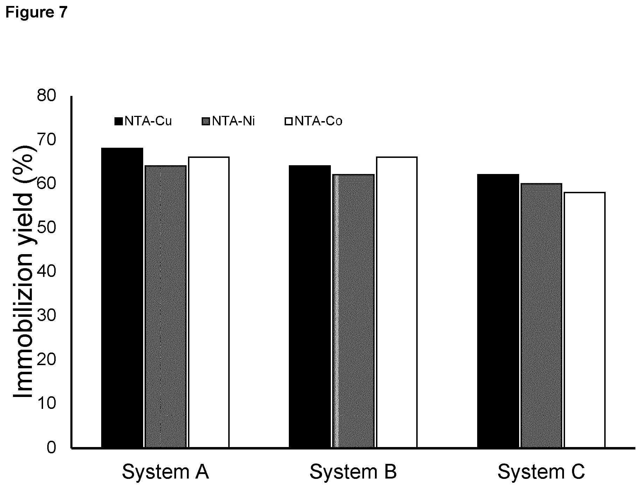

**Figure 7.** Immobilization yields of Green Fluorescence Protein (GFP) on MNPs functionalized with divalent metals. In all cases, 50 ug of protein was presented for each 0.1 mg of Fe. Measurements were made in triplicate. Since the deviation is less than 10%, it has not been represented in the graph. NTA-Cu (first column in each group), NTA-Ni (middle column), NTA-Co (right column)

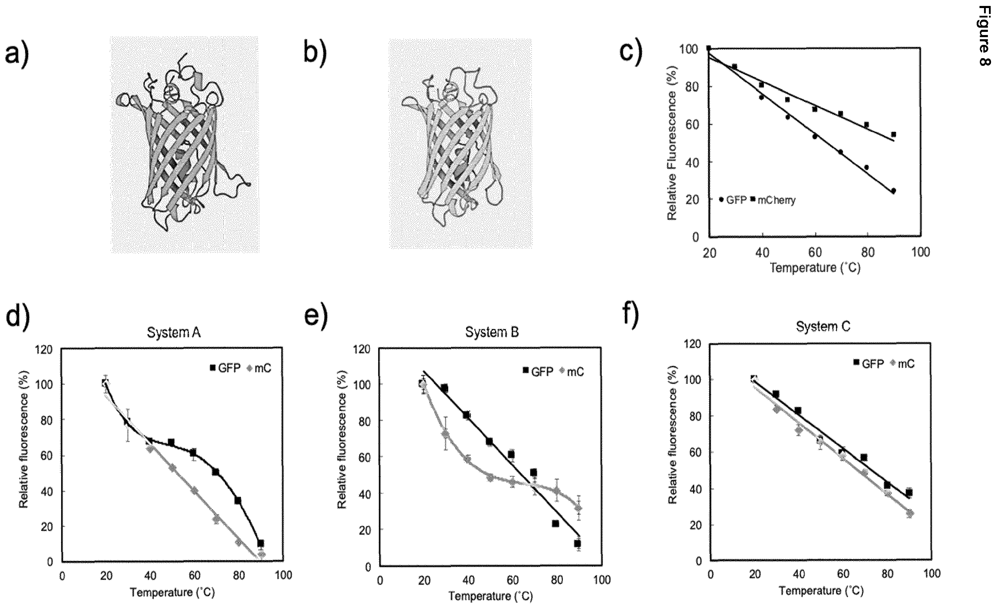

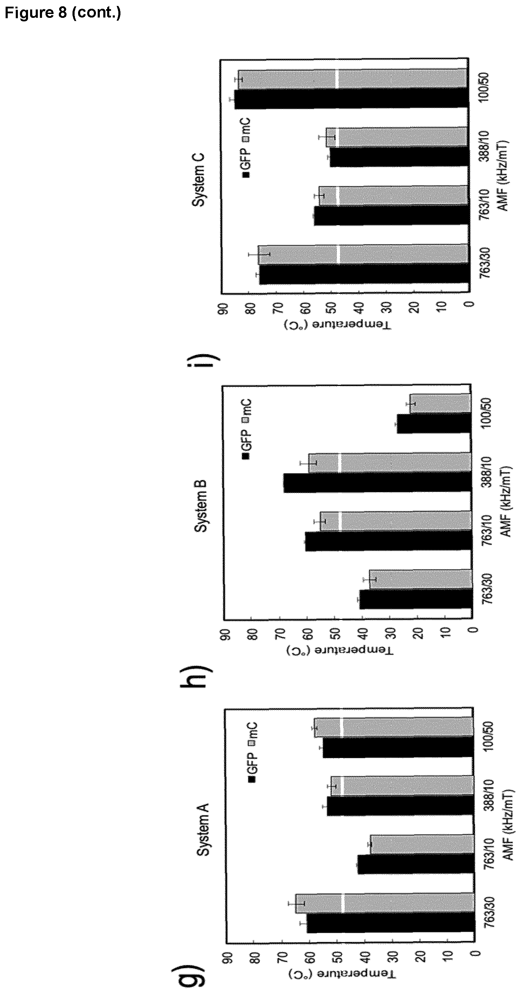

**Figure 8.** Temperature dependent fluorescence of the green fluorescent protein (GFP) and the m-Cherry Protein (mC) were used as thermal probes of the local temperature in the environment of MNPs. **A)** Ternary structure of the GFP **B)** Ternary structure of mC **C)** Fluorescent intensity dependence for free GFP and mC with temperature. Fluorescence intensity dependence with temperature for MNPs-GFP and MNPs-mC complexes: **D)** System A, **E)** System B and **F)** System C. Fluorescence reduction after exposure of MNPs-GFP/MNPs-mC complexes to different AMFs: **G)** System A, **H)** System B and **I)** System C.

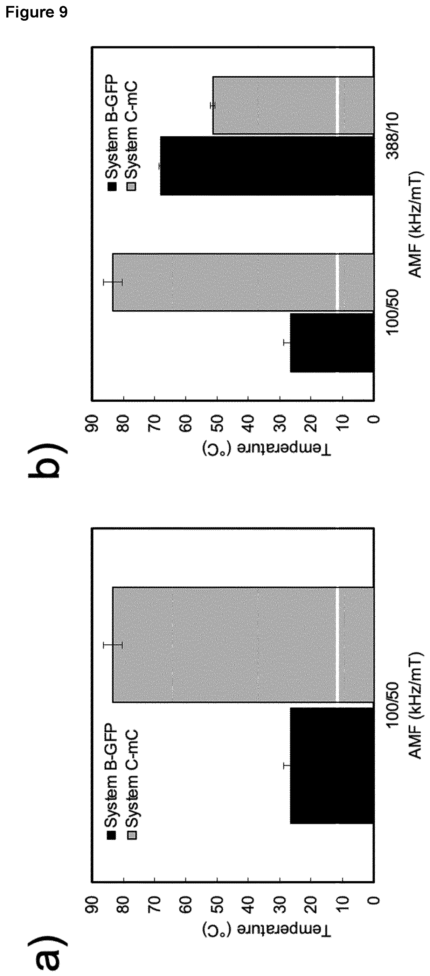

**Figure 9.** Generation of multiple local temperatures in a mixture of System B-GFP and System C-mC complexes exposed to AMF. GFP and mC fluorescence reductions were used as independent indicators of local temperatures. **A)** Temperatures registered for System B-GFP + System C-mC combination under 100 kHz and 50 mT AMF **B)** Temperatures registered for System B-GFP + System C-mC combination under different AMFs.

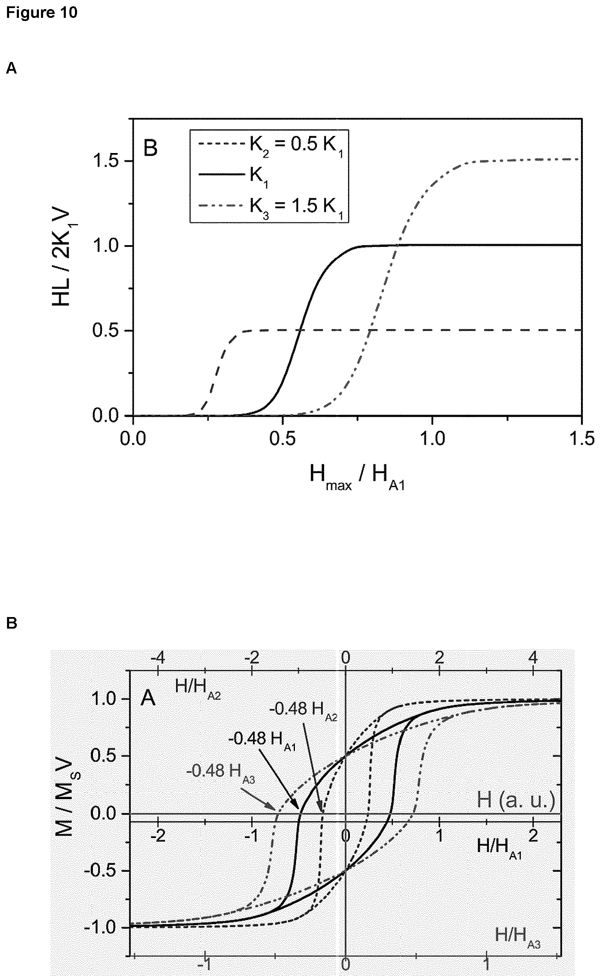

**Figure 10.** Theoretical hysteresis loops for non-interacting MNPs reported in Munoz-Menendez et al. [Munoz-Menendez et al. 2017] Towards improved magnetic fluid hyperthermia: major-loops to diminish variations in local heating Phys. Chem. Chem. Phys., 2017,19, 14527-14532 with permission from the PCCP Owner Societies. **A)** shows the M(H) magnetic loops of 3 different samples with the same characteristics (monodispersed, non and interacting, and the same $M_S$ and V values), the only difference being the value of $K_1$, $K_2 = 0.5K_1$ and $K_3 = 1.5K_1$. **(B)** Evolution of the corresponding global hysteresis losses, HL, as a function of $H_{max}$.

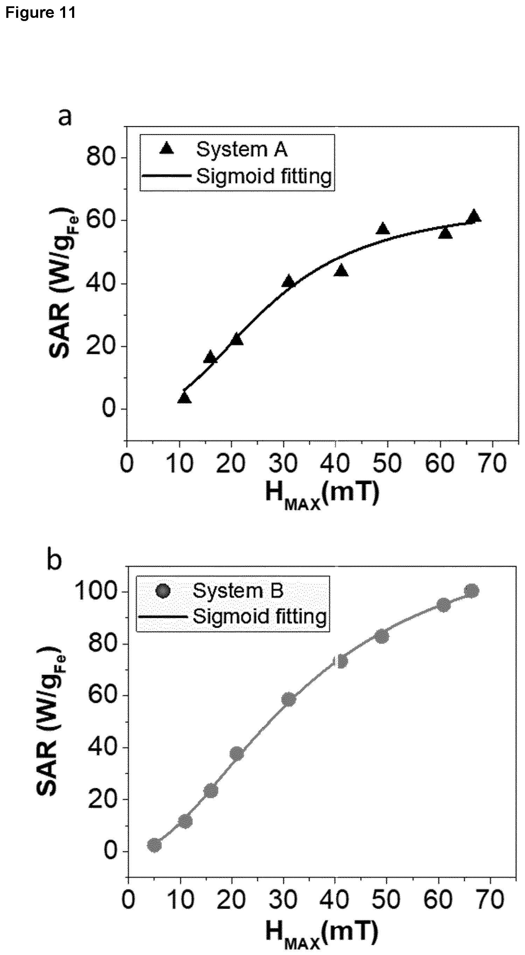

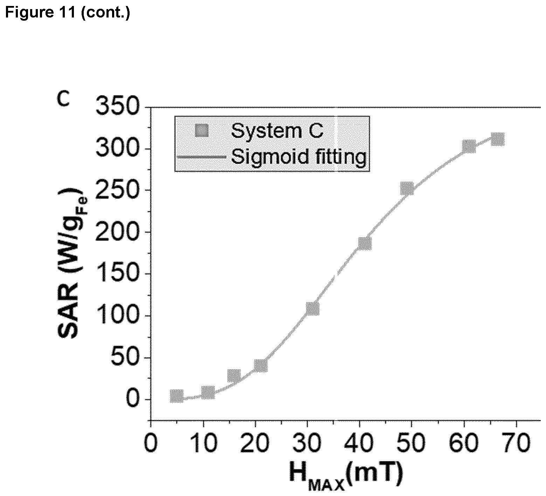

**Figure 11.** Sigmoidal fitting of SAR values dependence with $H_{max}$ presented in Figure 3b. **A)** System A, **B)** System B and **C)** System C.

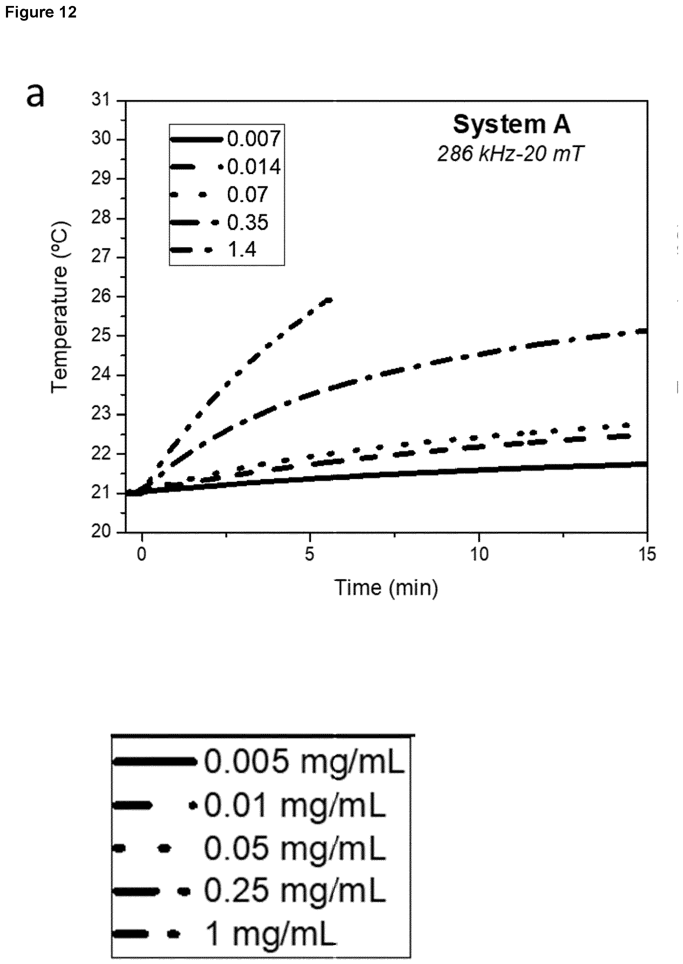

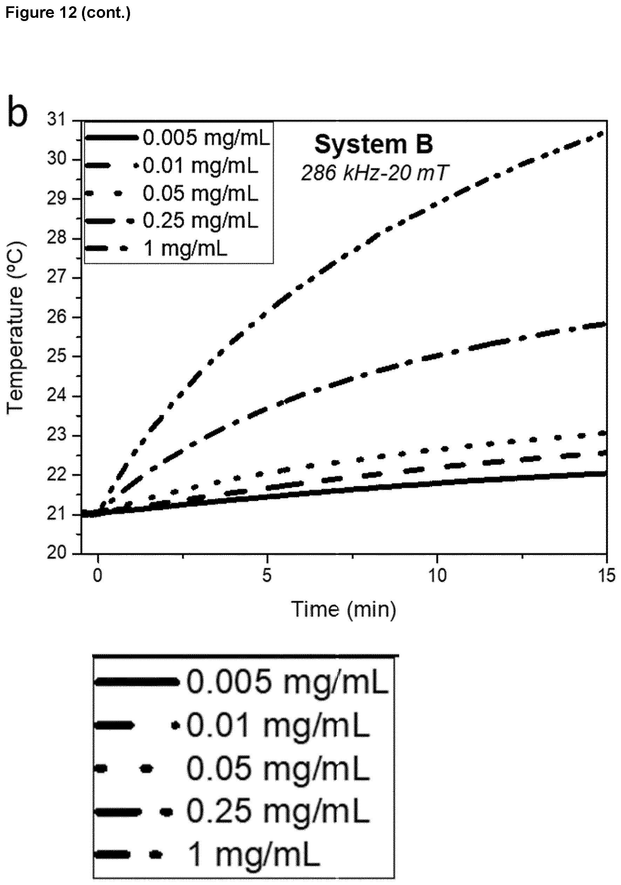

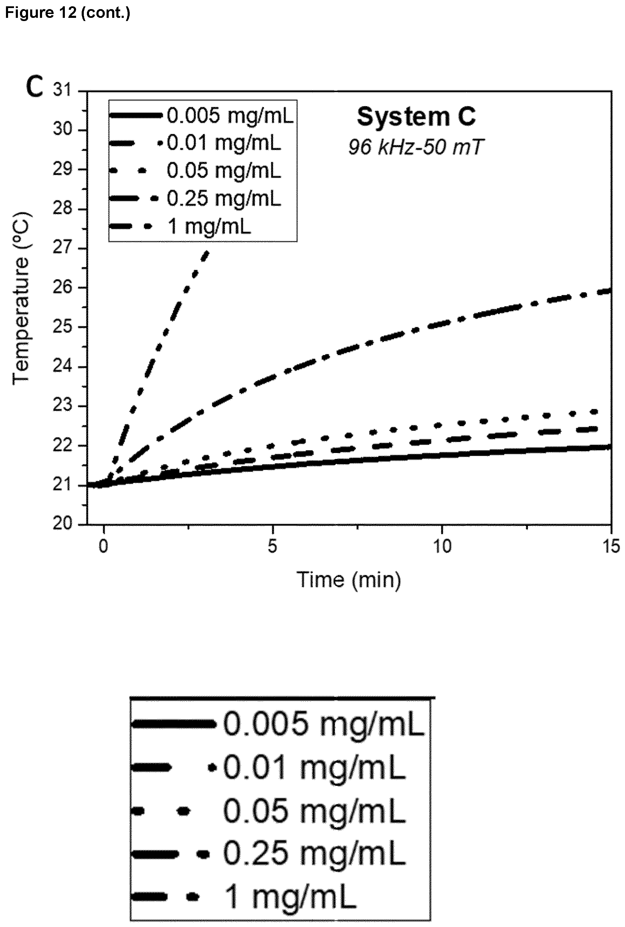

**Figure 12.** Increment of medium temperature with time in diluted colloids of: **A)** System A, **B)** System B and **C)** System C, at different MNPs concentrations and under the best corresponding AMF conditions.

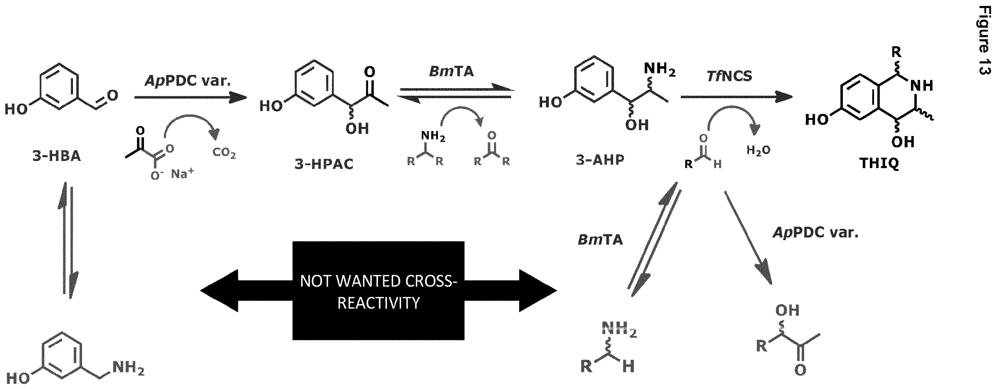

**Figure 13.** Example of on-demand sequential regulation of enzymes working together in a one-pot cascade system. Scheme of the three-enzyme cascade selected for the production of steropure isoquinolines. Abbreviations: i) ApPDC: *Acetobacter pasteurianus* pyruvate decarboxylase; ii) BmTA: *Bacillus megaterium* amino-transaminase; iii) TfNCS: *Thalictrum flavum* norcoclaurine synthase; iv) 3-HBA: 3-Hydroxybenzaldehyde, v) 3-HPAC: 3-Hydroxyphenylacetylcarbinol; vi) 3-HNP: 3-Hydroxynorephedrine; vii) THIQ: 1,3,4,-Trisubstituted tetrahydroisoquinolines.

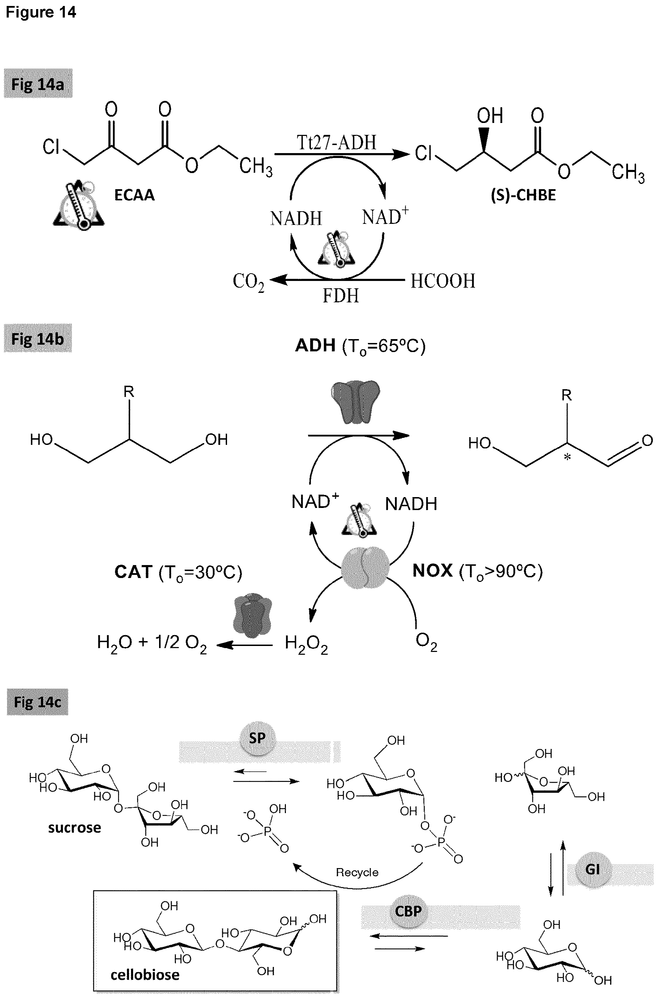

**Figure 14.** Examples of on-demand simultaneous activation of enzymes working together in a one-pot cascade system

- Figure 14a. Scheme of the bioprocess selected for the asymmetric reduction of a thermolabile β-ketoester by combining a thermophile (T27-ADH) and a mesophile (FDH) enzyme. Abbreviations: i) T27-ADH (alcohol dehydrogenase from *Thermus thermophilus HB27,* $T_{OPT}$ = 70°C); ii) FDH (formate dehydrogenase from *Candida boidinii,* $T_{OPT}$ = 37°C), iii) ECAA (Ethyl 4-chloroacetoacetate); iv) (S)-CHBE: Ethyl (S)-(-)-4-chloro-3-hydroxybutyrate; v) NADH (reduced form of nicotinamide adenine dinucleotide).

- Figure 14b. Scheme of the coupled multi-enzyme process selected for obtaining of hydroxy-propionaldehyde enantiopure derivatives by asymmetric oxidation of 1,3-propanediol derivatives. Abbreviations: i) ADH (alcohol dehydrogenase from B. *stearothermophilus,* $T_{OPT}$ = 65°C); ii) NOX (NADH-oxidase from *T. thermophilus,* $T_{OPT}$ = 90°C), iii) CAT (catalase from bovine liver, $T_{OPT}$ = 30°C), iv) NAD+ (nicotinamide adenine dinucleotide)
- Figure 14 c. Scheme of the three-enzyme cascade pathway selected for the synthesis of cellobiose from sucrose. Abbreviations: i) SP: sucrose phohphorylase from from L. *Mesenteroides* ($T_{OPT}$ =60°C), ii) GI: glucose isomerase from *S. murinus* ($T_{OPT}$ = 85°C), iii) CBP: cellobiose phosphorylase from ($T_{OPT}$ = 60°C).

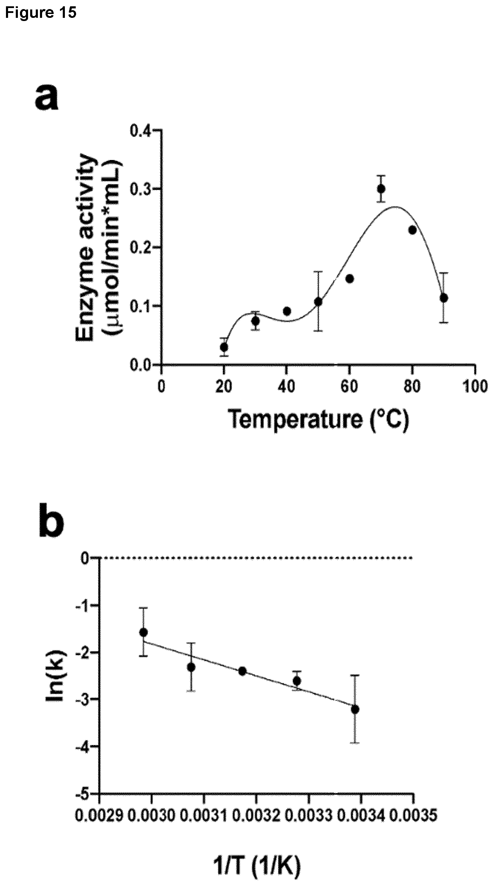

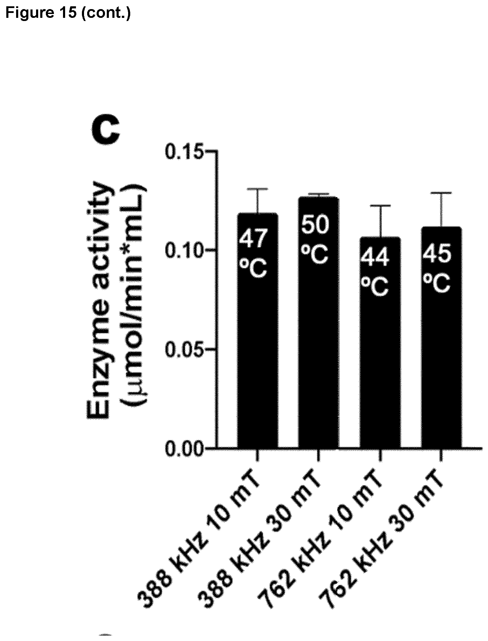

**Figure 15: a.** Activity vs temperature plot obtained by incubating 3.5 $\mu$g/mL of TtADH immobilized onto MNPs for 5 minutes at different temperatures. **b.** Arrhenius plot obtained from the Activity vs temperature plot. **c.** Enzymatic activity of 3.5 $\mu$g/mL of TtADH immobilized onto MNPs for 5 minutes under different AMF conditions, and inferred temperatures.

## BRIEF DESCRIPTION OF THE INVENTION

In a first aspect, the present invention relates to a process to carry out two or more enzymatic reactions in a reaction medium, comprising:

a) providing a system which in turn comprises at least two substantially homogeneous and colloidal populations of magnetic nanoparticles (MNPs), characterized in that each of the populations of MNPs are functionalized with at least one different enzyme,

characterized in that each of the populations of MNPs have a different magnetic anisotropy capable of dissipating local thermal energy on the surface of each of the populations of MNPs upon application of an external alternating magnetic field (AMF) sufficient to activate each of the enzymes functionalized on said MNPs, and

b) applying one or more external AMFs to the system of a) to produce a simultaneous or sequential activation of the enzymes functionalized on the surface of each of the populations of MNPs so that the enzymatic reactions are carried out,

wherein during step b) the reaction media is kept at a significantly lower temperature than the lowest optimum temperature of the enzymes functionalized on the surface of each of the populations of MNPs.

The process according to the first aspect or any of its embodiments, wherein the optimum temperature of the enzymes functionalized on each of the populations of MNPs is different in at least 10 °C.

The process of the first aspect or any of its embodiments, wherein the MNPs of each of the populations of MNPs of step a) are further characterized by having a particle-size distribution (PSD) of less than 0.25, preferably less than 0.1.

The process of the first aspect or any of its embodiments, wherein the MNPs of each of the populations of MNPs of step a) are further characterized by having an average particle diameter of less than 200 nm, preferably between 5 and 50 nm.

The process of the first aspect or any of its embodiments, wherein the MNPs of each of the populations of MNPs of step a) are further characterized by having a core selected from the group consisting of iron oxide, magnetite or maghemite.

The process of the first aspect or any of its embodiments, wherein the magnetic anisotropy capable of dissipating local thermal energy on the surface of each of the populations of MNPs is within the ranges of 34 mT $\pm$ 50%, preferably 34 mT $\pm$ 25%.

The process of the first aspect or any of its embodiments, wherein the MNPs of each of the populations of MNPs of step a) are further characterized by being coated with at least hydroxyl, carboxyl, carbonyl, amino, thiol, azide, or alkyne functional groups.

The process of the first aspect or any of its embodiments, wherein the coating is performed with polyacrylic acid (PAA), dimercaptosuccinic acid (DMSA) and/or poly(maleic anhydride-alt-1-octadecene (PMAO), or with any combination thereof.

The process of the first aspect or any of its embodiments, wherein the MNPs of each of the populations of MNPs of step a) are further characterized by being functionalized with at least a chelating agent and a divalent metal ion.

The process of the first aspect or any of its embodiments, wherein the chelating agent comprises nitriloacetic acid derivatives (NTA) and the divalent metal ions is selected from the group consisting of $Cu^{2+}$, $Co^{2+}$, $Ni^{2+}$.

The process of the first aspect or any of its embodiments, wherein the enzymes functionalized in the surface of the MNPs of each of the populations of MNPs of step a) are selected from the group consisting of Sucrose phosphorylase from *Leuconostoc mesenteroids,* Bacillus stearothermophilus alcohol dehydrogenase, *Thermus thermophilus* alcohol dehydrogenase, amylase from *Bacillus licheniformis,* glucose isomerase from *Suncus murinus,* cellobiose phosphorylase from *Clostridium thermocellum,* L-aspartate oxidase from *Sulfolobus tokodaii, Bacillus megaterium* or *Chromobacterium violaceum* transaminases, *Acetobacter pasteurianus* pyruvate decarboxylase, collagenase from *Bacillus sp,* hyaluronidase from *Thermasporomyces composti, Thermus thermophilus* lipase and NADH-oxidase, horseradish peroxidase, *Thermus thermophilus, Saccharolobus shibatae* uracil phosphoryl transferase, or *Escherichia coli* cytosine deaminase.

The process of the first aspect or any of its embodiments, wherein the magnetic anisotropy of each of the populations of MNPs is within the range of 34 mT ± 50%, and the external AMF is characterized by a frequency range between 50 and 800 kHz and a magnetic flux density of between 5 and 70 mT, wherein, preferably, the ratio between the magnetic flux density and the magnetic anisotropy is greater than 0.4, preferably greater than 0.5.

The process of the first aspect or any of its embodiments, wherein the magnetic anisotropy of each of the populations of MNPs is within the range of 34 mT ± 50%, and the external AMF is characterized by a frequency range 50 and 800 kHz and a magnetic flux density between 5 and 70 mT, and the ratio between the magnetic flux density and the magnetic anisotropy is between 0.4 and 1.2.

The process of the first aspect or any of its embodiments, wherein the magnetic anisotropy of each of the populations of MNPs is within the range of 34 mT ± 50%, and the external AMF is characterized by a frequency range between 50 and 800 kHz and a magnetic flux density of between 5 and 70 mT, and the ratio between the magnetic flux density and the magnetic anisotropy is between 0.5 and 1.1.

The process of the first aspect or any of its embodiments, wherein the process is to carry out two or more simultaneous enzymatic reactions, wherein the external AMFs of step b) is sufficient to produce the simultaneous activation of the enzymes functionalized on the surface of each of the populations of MNPs.

The process of the first aspect or any of its embodiments, wherein the process is to carry out two or more sequential enzymatic reactions, wherein the one or more applied external AMFs of step b) are applied sequentially so as to produce the sequential activation of the enzymes functionalized on each of the surfaces of each of the populations of MNPs.

The process of the first aspect or any of its embodiments, wherein the dissipated thermal energy on the surface of each of the at least two populations of MNPs is within the range of from 30-90° degrees.

The process of the first aspect or any of its embodiments, wherein the at least two substantially homogeneous and colloidal populations of magnetic nanoparticles (MNPs) comprised in the system of a) have a particle-size distribution of less than 0.5, preferably less than 0.1, have an iron oxide core and are selected from the group consisting of:

i) MNPs characterized in that each of the MNPs further have an average particle diameter of between 8-15 nm, are coated with DMSA, functionalized with NTA and at least a divalent ion, and have a magnetic anisotropy of 34 ± 25% mT;

ii) MNPs characterized in that each of the MNPs further have an average particle diameter of between 18-25 nm, are coated with PMAO, functionalized with NTA and at least a divalent ion, and have a magnetic anisotropy of 34 ± 25% mT; or

iii) MNPs characterized in that each of the MNPs further have an average particle diameter of between 25-35 nm, are coated with PAA, functionalized with NTA and at least a divalent ion, and have a magnetic anisotropy of 34 ± 25% mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz,
v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

The process of the first aspect or any of its embodiments, wherein the at least two substantially homogeneous and colloidal populations of magnetic nanoparticles (MNPs) comprised in the system of a) have a particle-size distribution of less than 0.1, have an iron oxide core and are selected from the group consisting of:

i) MNPs characterized in that each of the MNPs further have an average particle diameter of between 8-15 nm, are coated with DMSA, functionalized with NTA and at least a divalent ion, wherein the divalent metal ion is selected from the group consisting of $Cu^{2+}$, $Co^{2+}$, $Ni^{2+}$, and have a magnetic anisotropy of 34 ± 25% mT;

ii) MNPs characterized in that each of the MNPs further have an average particle diameter of between 18-25 nm, are coated with PMAO, functionalized with NTA and at least a divalent ion, wherein the divalent metal ion is selected from the group consisting of $Cu^{2+}$, $Co^{2+}$, $Ni^{2+}$, and have a magnetic anisotropy of 34 ± 25% mT; or

iii) MNPs characterized in that each of the MNPs further have an average particle diameter of between 25-35 nm, are coated with PAA, functionalized with NTA and at least a divalent ion, wherein the divalent metal ion is selected from the group consisting of $Cu^{2+}$, $Co^{2+}$, $Ni^{2+}$, and have a magnetic anisotropy of 34 ± 25% mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz,
v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

The process of the first aspect or any of its embodiments, wherein the at least two substantially homogeneous and colloidal populations of magnetic nanoparticles (MNPs) comprised in the system of a) have a particle-size distribution of less than 0.1, have an iron oxide core and are selected from the group consisting of:

i) MNPs characterized in that each of the MNPs further have an average particle diameter of between 8-15 nm, are coated with DMSA, functionalized with NTA and $Cu^{2+}$, and have a magnetic anisotropy of 27 mT;
ii) MNPs characterized in that each of the MNPs further have an average particle diameter of between 18-25 nm, are coated with PMAO, functionalized with NTA and $Cu^{2+}$, and have a magnetic anisotropy of 34 mT; or
iii) MNPs characterized in that each of the MNPs further have an average particle diameter of between 25-35 nm, are coated with PAA, functionalized with NTA and $Cu^{2+}$, and have a magnetic anisotropy of 41 mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz,
v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

In a second aspect, the present invention relates to a system comprising at least two substantially homogeneous and colloidal populations of magnetic nanoparticles (MNPs), characterized in that each of the populations of MNPs are functionalized with at least one different enzyme, and characterized in that each of the populations of MNPs have a different magnetic anisotropy capable of dissipating local thermal energy on the surface of each of the population of MNPs upon application of an external alternating magnetic field (AMF) sufficient to activate each of the enzymes functionalized on said MNPs.

The system according to the second aspect or any of its embodiments, wherein the optimum temperature of the enzymes functionalized on each of the populations of MNPs is different in at least 10 °C.

The system of the second aspect or any of its embodiments, wherein the MNPs of each of the populations of MNPs of step a) are further characterized by having a particle-size distribution (PSD) of less than 0.25, preferably less than 0.1.

The system of the second aspect or any of its embodiments, wherein the MNPs of each of the populations of MNPs of step a) are further characterized by having an average particle diameter of less than 200 nm, preferably between 8 and 50 nm.

The system of the second aspect or any of its embodiments, wherein the MNPs of each of the populations of MNPs of step a) are further characterized by having a core selected from the group consisting of iron oxide, magnetite or maghemite.

The system of the second aspect or any of its embodiments, wherein the magnetic anisotropy capable of dissipating local thermal energy on the surface of each of the populations of MNPs is within the ranges of 34 mT ± 50%, preferably 34 mT ± 25%.

The system of the second aspect or any of its embodiments, wherein the MNPs of each of the populations of MNPs of step a) are further characterized by being coated with at least hydroxyl, carboxyl, carbonyl, amino, thiol, azide, or alkyne functional groups.

The system of the second aspect or any of its embodiments, wherein the coating is performed with polyacrylic acid (PAA), dimercaptosuccinic acid (DMSA) and/or poly(maleic anhydride-alt-1-octadecene (PMAO), or with any combination thereof.

The system of the second aspect or any of its embodiments, wherein the MNPs of each of the populations of MNPs of step a) are further characterized by being functionalized with at least a chelating agent and a divalent metal ion.

The system of the second aspect or any of its embodiments, wherein the chelating agent comprises nitriloacetic acid derivatives (NTA) and the divalent metal ions is selected from the group consisting of $Cu^{2+}$, $Co^{2+}$, $Ni^{2+}$.

The system of the second aspect or any of its embodiments, wherein the enzymes functionalized in the surface of the MNPs of each of the populations of MNPs of step a) are selected from the group consisting of Leuconostoc mesenteroids, *Bacillus stearothermophilus* alcohol dehydrogenase, *Thermus thermophilus* alcohol dehydrogenase, amylase from *Bacillus licheniformis,* glucose isomerase from *Suncus murinus,* cellobiose phosphorylase from *Clostridium thermocellum,* L-aspartate oxidase from *Sulfolobus tokodaii, Bacillus megaterium* or *Chromobacterium violaceum* transaminases, *Acetobacter pasteurianus* pyruvate decarboxylase, collagenase from *Bacillus sp,* hyaluronidase from *Thermasporomyces composti, Thermus thermophilus* lipase and NADH-oxidase, horseradish peroxidase, *Thermus thermophilus, Saccharolobus shibatae* uracil phosphoryl transferase, or *Escherichia coli* cytosine deaminase.

The system of the second aspect or any of its embodiments, wherein the at least two substantially homogeneous and colloidal populations of magnetic nanoparticles (MNPs) comprised in the system of a) have a particle-size distribution of less than 0.5, preferably less than 0.1, have an iron oxide core and are selected from the group consisting of:

   i) MNPs characterized in that each of the MNPs further have an average particle diameter of between 8-15 nm, are coated with DMSA, functionalized with NTA and at least a divalent ion, and have a magnetic anisotropy of 34 $\pm$ 25% mT;
   ii) MNPs characterized in that each of the MNPs further have an average particle diameter of between 18-25 nm, are coated with PMAO, functionalized with NTA and at least a divalent ion, and have a magnetic anisotropy of 34 $\pm$ 25% mT; or
   iii) MNPs characterized in that each of the MNPs further have an average particle diameter of between 25-35 nm, are coated with PAA, functionalized with NTA and at least a divalent ion, and have a magnetic anisotropy of 34 $\pm$ 25% mT.

The system of the second aspect or any of its embodiments, wherein the at least two substantially homogeneous and colloidal populations of magnetic nanoparticles (MNPs) comprised in the system of a) have a particle-size distribution of less than 0.1, have an iron oxide core and are selected from the group consisting of:

   i) MNPs characterized in that each of the MNPs further have an average particle diameter of between 8-15 nm, are coated with DMSA, functionalized with NTA and at least a divalent ion, wherein the divalent metal ion is selected from the group consisting of $Cu^{2+}$, $Co^{2+}$, $Ni^{2}$, and have a magnetic anisotropy of 34 $\pm$ 25% mT;
   ii) MNPs characterized in that each of the MNPs further have an average particle diameter of between 18-25 nm, are coated with PMAO, functionalized with NTA and at least a divalent ion, wherein the divalent metal ion is selected from the group consisting of $Cu^{2+}$, $Co^{2+}$, $Ni^{2+}$, and have a magnetic anisotropy of 34 $\pm$ 25% mT; or
   iii) MNPs characterized in that each of the MNPs further have an average particle diameter of between 25-35 nm, are coated with PAA, functionalized with NTA and at least a divalent ion, wherein the divalent metal ion is selected from the group consisting of $Cu^{2+}$, $Co^{2+}$, $Ni^{2+}$, and have a magnetic anisotropy of 34 $\pm$ 25% mT.

The system of the second aspect or any of its embodiments, wherein the at least two substantially homogeneous and colloidal populations of magnetic nanoparticles (MNPs) comprised in the system of a) have a particle-size distribution of less than 0.1, have an iron oxide core and are selected from the group consisting of:

   i) MNPs characterized in that each of the MNPs further have an average particle diameter of between 8-15 nm, are coated with DMSA, functionalized with NTA and $Cu^{2+}$, and have a magnetic anisotropy of 27 mT;
   ii) MNPs characterized in that each of the MNPs further have an average particle diameter of between 18-25 nm, are coated with PMAO, functionalized with NTA and $Cu^{2+}$, and have a magnetic anisotropy of 34 mT; or
   iii) MNPs characterized in that each of the MNPs further have an average particle diameter of between 25-35 nm, are coated with PAA, functionalized with NTA and $Cu^{2+}$, and have a magnetic anisotropy of 41 mT.

The system of the second aspect or any of its embodiments, wherein the system further comprises a device suitable for generating an external AMF.

*In vitro* use of the system according to the second aspect or any of its embodiments, to carry out the enzymatic process according to the first aspect or any of its embodiments.

## DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus ten percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of" does not exclude materials or steps that do not materially affect the basic and novel characteristics of the claim.

By "magnetic anisotropy" (K) is meant herein the property that confers a preferred direction on the spins of a system, which may not be aligned with an external magnetic field. This definition is further developed below.

"Superparamagnetism" refers to materials that do not exhibit magnetic remanence when no magnetic field is applied but behave similar to a ferromagnetic material upon the application of a magnetic field.

By "coating" or "functionalization" in the context of the present invention is meant the modification of the surface of the MNPs with the application of at least one layer of a material giving the surface new properties and/or functionalities.

By "Optimum temperature" (OT) of an enzyme is referred to the temperature range in which a maximal rate of reaction is achieved.

### DESCRIPTION

Most of the nanoparticle uses as nanoheaters seek to optimize processes by maximizing the amount of heat dissipated, since most of the final purposes of MNPs in biomedicine are directed to kill tumours, achieving a global temperature increase of the tumour microenvironment that is known as hyperthermia assays.

However, when it comes to the use of MNPs and their heating properties for tuning enzymatic processes, the production of heat requires an exquisite control of the temperature, since too high or too low temperatures can be detrimental for the activity of the enzyme or even for its structure. This becomes even more difficult when a one-pot system is required, that is, when several MNPs functionalized with several enzymes operate in the same pot, sharing space. Currently available systems do not allow adjusting the degree of heating generated locally by the nanoparticles, let alone controlling the activation of more than one enzyme in more than one population of MNPs in a one-pot setting. This is key in industrial multi-enzymatic processes, where the reactions may be carried out by several enzymes that need to work sequentially or simultaneously in the same reaction vessel.

In the present invention, this problem is solved by a deep analysis and fine-tuning of the required properties of the MNPs used and of the external alternating magnetic field (AMF) applied. The authors describe herein the main characteristics that impact the thermal energy dissipated by the surface of the MNPs, and the conditions of the AMF that need to be applied in order to achieve a precise control of the thermal energy produced that will allow the enzymes to work at optimal temperatures on the surface of the MNPs, allowing the combination of different MNPs population with different enzymes in a one-pot system. This is possible by using a combination of magnetic nanoparticles with different magnetic anisotropy (which defines their structural and physicochemical characteristics) and specific AMFs with variable frequencies and intensities.

The populations of MNPs selected are characterized in that they induce different "local temperatures" on their surface upon application of an external AMF. This is possible because each of the populations of MNPs have different magnetic anisotropy and behave differently upon a specific external AMF. The local temperature achieved in each case is closely related to the hysteresis losses of the MNPs when exposed to an external AMF. Thus, in the present invention, the anisotropy field of the MNPs is proposed as a global parameter to define the heating potential of the different MNPs upon application of one or more external AMFs.

The amount of heat produced by a colloid of magnetic nanoparticles (MNPs) can be derived from their magnetic loops. The energy dissipated is generally defined as specific absorption rate (SAR) and it can be derived from the product of the area inside the cycle (A) times the frequency of the applied field (f).

$$SAR = A\,f \approx 2\,K\,V\,f$$

Thus, for any magnetic system it is true that the hysteresis losses will be maximized by increasing the area of the hysteresis loops and the frequency of the applied field.

As shown in Figure 3, by tailoring the magnetic properties of the MNPs it is possible to generate different SAR and different dependence of SAR with AMF conditions. For example, at similar AMF conditions of 92 kHz/50 mT (Figure 3a), the SAR is maximum in the case of highly anisotropic MNP (System C) and minimum in the case of MNPs with low anisotropy (System A).

Further, it is also shown herein that the MNPs can be efficiently functionalized with different enzymes and chemical compounds or coatings without compromising their key properties that allow the fine control of the thermal dissipation (see, e.g., Figures 6 and 8).

In conclusion, through extensive study of MNPs and their behaviour upon application of different AMFs, the inventors have found a methodology to carry out two or more enzymatic reactions in a single reaction medium to produce a simultaneous or sequential activation and/or inactivation of the enzymes functionalized on the surface of each of the populations of MNPs so that the enzymatic reactions are carried out. The present invention also describes a panel of MNPs with different anisotropy that in combination with certain AMF settings is possible to carry out two or more enzymatic reactions in one-pot configuration.

In **a first aspect**, the invention relates to a process to carry out two or more enzymatic reactions in a reaction medium, comprising:

a) providing a system which in turn comprises at least two substantially homogeneous and colloidal populations of magnetic nanoparticles (MNPs), characterized in that each of the populations of MNPs are functionalized with at least one different enzyme,
characterized in that each of the populations of MNPs have a different magnetic anisotropy capable of generating different local temperatures on the surface of each of the populations of MNPs upon application of an external alternating magnetic field (AMF) sufficient to activate and/or inactivate each of the enzymes functionalized on said MNPs, and
b) applying one or more external AMFs to the system of a) to produce a simultaneous or sequential activation and/or inactivation of the enzymes functionalized on the surface of each of the populations of MNPs so that the enzymatic reactions are carried out,

wherein during step b) the reaction media is kept at a significantly lower temperature than the lowest optimum temperature of the enzymes functionalized on the surface of each of the populations of MNPs.

In a preferred embodiment, the process is carried out in a one-pot reaction medium.

Preferably, but not limiting the scope of the present invention, the activation and/or inactivation of an enzyme is a "partial or total activation and/or inactivation of an enzyme", which is understood as an increase or decrease in the rate of the catalytic reaction carried out by an enzyme in comparison to the catalytic reaction rate of said enzyme when measured at the temperature of the reaction media prior to the application of an external alternating magnetic field. More preferably, said increase in the catalytic reaction rate provides for a catalytic reaction rate in the range of at least 10%, 20%, 30%, 40%, 50%, 60%, 70%, 80%, 90% or more of the highest enzymatic catalytic activity reachable for that particular enzyme under its optimum conditions (temperature, substrate, cofactors, etc), as long as said increase provides a superior catalytic reaction rate to the catalytic reaction rate of said enzyme when measured at the temperature of the reaction media. Also preferably, said decrease in the catalytic reaction rate provides for a catalytic reaction rate in the range of at least 0%, 10%, 20%, 30%, 40%, 50%, 60%, 70%, 80%, 90% or more of the highest enzymatic catalytic activity reachable for that particular enzyme under its optimum conditions (temperature, substrate, cofactors, etc), as long as said decrease provides an inferior catalytic reaction rate to the catalytic reaction rate of said enzyme when measured at the temperature of the reaction media. Still more preferably, said increase or decrease can be a gradual increase or decrease. Changes of the rate of the catalytic reaction of an enzyme may be quantified by measuring the rate of change in concentration of the substrate that the enzyme uses for its catalytic activity.

Each of the clauses of the first aspect (steps a), b), and the clause referring to the reaction media) are explained in detail below. It is noted that all the embodiments comprised in each of the clauses can be combined among them and with any other embodiments comprised in any other different clause. It is thus understood that any possible combination between embodiments from the same and from different clauses is also encompassed in the present invention.

With respect to the step a) of the first aspect, the following embodiments are comprised:

In an embodiment according to the first aspect, a system comprising at least two substantially homogeneous and colloidal populations of magnetic nanoparticles (MNPs) is provided.

"Magnetic nanoparticles" (MNPs) as referred herein are a class of nanoparticles that can be manipulated using magnetic fields. The system according to the first aspect of the present invention or any of its embodiments may comprise two or more populations of MNPs. In an embodiment, the system may comprise two, three, four, or five populations of MNPs. By "population of MNPs" is meant a plurality MNPs that are homogeneous in their sizes and shapes, and that form a colloidal dispersion. By "homogeneous" is meant that the particles in each of the populations of MNPs according to the first aspect are characterized by having substantially the same particle size or diameter, that is, for being substantially monodisperse. As used herein, "monodisperse" refers to MNPs possessing a narrow average particle size distribution (PSD). By "particle size distribution" (PSD) is meant herein an index (means of expression) indicating what sizes (particle size) of particles are present in what proportions (relative particle amount as a percentage where the total amount of particles is 100 %) in the sample particle group to be measured. Preferably, particle size distribution can be measured from a statistically valid analysis of hundreds of particles from TEM images, and it refers to the standard deviation of the fitting. By "colloidal" is meant that the plurality of MNPs of each of the populations of MNPs remain suspended in solution at equilibrium, that is, they do not tend to aggregate or flocculate.

In an embodiment, the plurality of MNPs are at least 70%, 80%, 90%, 95%, 96%, 97%, 98%, 99%, or 100% identical among them in a population of MNPs. By "identical" is meant that the MNPs are substantially similar in their characteristics, including shape, composition, magnetic properties, coating and functionalization. Preferably, the MNPs of each of the populations of MNPs according to the first aspect of the present invention or any of its embodiments are at least 85%, 90%, 92%, 95%, 98%, 100% identical among them, more preferably 95% identical.

In an embodiment, the MNPs of each of the populations of MNPs according to the first aspect of the present invention or any of its embodiments or any of its embodiments have a particle size distribution of less than 0.5, 0.4, 0.3, 0.25, 0.2, preferably less than 0.1, or 0.05. The particle size and PSD of nanoparticles can be determined using numerous commercially available instruments and methods known by the skilled artisan. An example of this method is photon correlation spectroscopy (PCS) that the dynamic fluctuation of the scattered light intensity is the basis for calculation of the average particle size.

In an embodiment, each MNP comprises a magnetic core and an organic coating outer shell, in which is the outer shell that plays a threefold role: it protects the core from dissolution, enhances magnetic properties of the nanoparticle by keeping them apart and introduces an anchoring point for enzyme conjugation.

The core of the MNPs may be made of different inorganic materials. In an embodiment the core of MNPs is made of pure inorganic materials, such as Fe, Co or Ni; metal oxides such as iron oxide, or metal alloys such as FePt and CoPt. In an embodiment, the core of the MNPs can include Fe, Co, Ni, FePt, or SmCo. More preferably, the core of the MNPs is a ferromagnetic core selected from the group consisting of iron oxide, magnetite or maghemite. In some embodiments, the iron oxide core includes a dopant material. The dopant material can include a metal selected from the group consisting of Mn, Co, Ni, Zn, and ZnMn.

In a preferred embodiment, the MNPs of each of the populations of MNPs of step a) of the first aspect of the present invention are characterized by having a particle size distribution of less than 0.1, and have a core selected from the group consisting of iron oxide, magnetite or maghemite.

In an embodiment, the average particle diameter of the core of the MNP ranges from 1 to 200 nm. In another embodiment, the average particle diameter of the MNP ranges from 5 and 50 nm. Preferably, the average particle diameter of less than 200 nm, preferably between 8 and 50 nm. More preferably, the average particle diameter of the MNPs may be less than or equal to about 50 nm, 40 nm, 30 nm, 20 nm, 10 nm or 5 nm. In another embodiment, the average particle diameter ranges from 5-50 nm, 5-40 nm, 5-30 nm, 5-20 nm, 8-15 nm, 18-25 nm, 25-35 nm or any value within these ranges. In another embodiment, the average particle diameter of the MNP is at least 5 nm, 6 nm, 7 nm, 8 nm, 9 nm, 10 nm, 11 nm, 12 nm, 13 nm, 14 nm, 15 nm, 16 nm, 17 nm, 18 nm, 19 nm, 20 nm, 21 nm, 22 nm, 23 nm, 24 nm, 25 nm, 26 nm, 27 nm, 28 nm, 29 nm, 30 nm, 31 nm, 32 nm, 33 nm, 34 nm or 35 nm.

In an embodiment, the MNPs of each of the populations of MNPs can have different shapes, such as spherical, cubic, prism, rod, tubes, cylindrical, squared, etc.

In an embodiment, the MNPs of each of the population of MNPs are in a concentration within the range of 0.0001-3 mg of Fe/mL. In a further embodiment, MNPs are in a concentration within 0.0001-0.25 mg of Fe/mL. In further embodiment, MNPs are in a concentration within 0.0001-0.1 mg of Fe/mL. In another embodiment, the MNPs are in a concentration equal or greater than 0.25, 0.05 or 0.005 mg of Fe/mL.

Methods to synthesize magnetic nanoparticles of the desired size are known in the art. In an embodiment, the methods for generating the MNPs are selected from precipitation, microemulsion, thermal decomposition, solvothermal, sonochemical, microwave and assisted, chemical vapor deposition, combustion, carbon arc, laser pyrolysis, electrochemical synthesis, microorganism or bacterial synthesis. In a preferred embodiment, the methods used herein to synthetize the MNPs are selected from coprecipitation in aqueous media, thermal decomposition in organic media or oxidative precipitation in aqueous media.

Further, in an embodiment, the populations of MNPs according to the first aspect of the present invention or any of its embodiments have different magnetic anisotropy. The magnetic anisotropy "K" is meant herein the property that confers a preferred direction on the spins of a system, which may not be aligned with an external magnetic field. In the base of this parameter the anisotropy field "$H_A$" is defined as:

$$H_A = 2K/M_S$$

being K the anisotropy constant of the system and $M_S$ the saturation magnetization, intrinsic properties of the material depending on the chemical and crystalline nature, the particle size and shape, and aggregation state. The $H_A$ is commonly used to define the limit at which the MNPs dissipate heat or not, and it is of primary importance in determining the magnetic properties of particle systems, because it weights the relative importance of the Zeeman energy.

In the present invention, the $H_A$ reference value of each of the population of MNPs is chosen as 34 mT. Thus, in the context of the present invention, the $H_A$ of each of the population of MNPs may be higher or lower than 34 mT. In an embodiment, the $H_A$ of each of the population of MNPs is within the range of 34 mT $\pm$ 60%, 34 mT $\pm$ 50%, 34 mT $\pm$ 40%, 34 mT $\pm$ 30%, 34 mT $\pm$ 25%, 34 mT $\pm$ 20%, or 34 mT $\pm$ 10%. More preferably, the $H_A$ of each of the population of MNPs is within the range of 34 mT $\pm$ 25%. In an embodiment, the $H_A$ of each of the population of MNPs is selected from the group consisting of $H_A$ of about 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, **44, 45,** 46, 47, 48, 49, 50, 51, 52, 53, 54, 55, 56, 57, 58, 59, or 60 mT. In a preferred embodiment, the $H_A$ of each of the population of MNPs is selected from the group consisting of $H_A$ of 27 mT $\pm$ 25%, 34 mT $\pm$ 25%, or 41 mT $\pm$ 25%. In a further preferred embodiment, the $H_A$ of each of the population of MNPs is selected from the group consisting of $H_A$ of 27 mT, 34 mT, or 41 mT. In another preferred embodiment, the system comprises at least two population of MNPs characterized by having a core of iron oxide and further characterized by having a magnetic anisotropy selected from the group consisting of 27 mT, 34 mT, or 41 mT (from hereinafter said MNPs populations shall be referred to as systems A , B and C, respectively).

For a randomly oriented set of MNPs, the maximum magnetic loop area is $\approx$2KV, with K the particle anisotropy constant and V its volume. Considering this, the equation above indicates that the amount of heat dissipated can be increased by increasing the frequency, or alternatively by using MNPs with higher anisotropy. But this is only true if the intensity of the applied field is high enough to overcome the particle anisotropy. Otherwise, the MNPs with high anisotropy only reproduce minor magnetic loops and the amount of heat generated is lower than the one of MNPs with smaller K.

MNPs with excellent bulk physical and chemical properties may not possess suitable surface properties for specific applications that are conferred by the shell. For this reason, surface coating and modification techniques that can transform these materials into valuable finished products are an important part of nanomaterials. In an embodiment, the MNPs may be surface coated to improve their stability and/or to make them biocompatible for further functionalizations such as, for example, with enzymes. By "coating" or "functionalization" is meant the modification of the surface of the MNPs with the application of at least one layer of a material (shell) giving the surface new properties and/or functionalities. The surface coating or the surface functionalization can be realized with more than one different compound, such as with metal ions, small molecules, surfactants, or polymers first and then with a second layer of a chelating agent.

Thus, in an embodiment, the MNPs of each of the populations of MNP are surface coated.

In an embodiment, the shell has a thickness ranging from 0.1 nm to 20 nm. In a further embodiment, the shell has a thickness ranging from 1 to 20 nm. In further embodiment, the shell has a thickness of 1, 2, 3, 4, 5, 6, 7, 8, 9, 10, 11, 12, 13, 14, 15, 16, 17, 18, 19 or 20 nm.

In an embodiment, the MNPs of each of the populations of MNPs are further characterized by being coated with molecules that comprise hydroxyl, carboxyl, carbonyl, amino, thiol, azide, or alkyne functional groups. In a preferred embodiment, the MNPs of each of the populations of MNPs of step a) are further characterized by being coated with at least hydroxyl, carboxyl, carbonyl, amino, thiol, azide, or alkyne functional groups. In an embodiment, the MNPs are coated with functional molecules. In an embodiment, the molecules are selected from the group consisting of carboxyl molecules when particles are prepared in aqueous media and amphiphilic molecules when particles are prepared in organic media. In a preferred embodiment, the MNPs are coated with polyacrylic acid (PAA), oleic acid, dimercaptosuccinic acid (DMSA) and/or poly(maleic anhydride-alt-1-octadecene (PMAO), any compound derived thereof, or any combination thereof.

In an embodiment, the concentration of the carboxylic, hydroxylic and/or aminated functional groups coated in the MNPs of each of the populations of MNPs is at a concentration range of around 50% respect to the mass of inorganic cores. In a preferred embodiment, the concentration of the chelating agent coated in the MNPs of each of the populations of MNPs is at a mass percentage in the range of 5-20% respect to the mass of inorganic cores.

In an embodiment, the coating with PAA, PMAO and DMSA acid provides the colloidal stability at a wide pH range and the possibility to anchor the chelating agent in order to bind his-tagged recombinant proteins by metal affinity binding.

In another embodiment, the MNPs of each of the populations of MNP are further characterized by being functionalized with at least a chelating agent anchored to the surface of the MNPs. In an embodiment, at least a chelating agent further comprises at least a divalent metal ion ($Me^{2+}$). In a preferred embodiment, the at least a divalent metal ion is selected from the group consisting of a divalent metal ion is selected from the group consisting of $Cu^{2+}$, $Co^{2+}$, or $Ni^{2+}$. In a preferred embodiment, the chelating agent comprises nitriloacetic acid derivatives (NTA), and the at least a divalent metal ion is selected from the group consisting of $Cu^{2+}$, $Co^{2+}$, or $Ni^{2+}$.

In an embodiment, the concentration of the chelating agent coated on the MNPs of each of the populations of MNPs is at a concentration range of 50 $\mu$M $\pm$ 50%. In a preferred embodiment, the concentration of the chelating agent coated in the MNPs of each of the populations of MNPs is at a concentration range of 0.05 $\mu mol_{NTA-Me}$ / $mg_{Fe}$ $\pm$ 50%.

Methods to coat or functionalize MNPs with biomolecules (drugs, proteins, enzymes, antibodies, or nucleotides) are also known in the art. In an embodiment, the MNPs of each of the populations according to the first aspect of the present invention or any of its embodiments are functionalized with enzymes. In an embodiment, the methods for coating the MNPs are selected among amine reactive chemistries (e.g. cyanogen bromide, N-hydroxy succinimide esters, reductive amination, carbodiimide-mediated bond formation, etc), sulfhydryl reactive chemistries (eg. maleimide, pyridyl disulfide, epoxy activation, etc,), carbonyl reactive chemistries (hydrazide, reductive amination), hydroxyl reactive chemistries (epoxy activation chemistries, divinylsulfone, cyanuric chloride, etc), metal affinity based-conjugation. In a preferred embodiment, metal affinity based-conjugation is used for site-specific binding of his-tagged recombinant enzymes by the use of immobilized metal ions (such as cobalt, nickel, copper, zinc) by previously anchoring chelating agents (such as nitrile acetic acid) to the MNP coating. In a preferred embodiment, cobalt and copper are used as immobilized metal ions.

In an embodiment, metal affinity based enzyme immobilization conditions are known by the skilled artisan being able to be carried out in a broad range of pH (5-9, being 6 to 8 usually preferred binding pHs) and buffers (Na-phosphate, phosphate citrate, HEPES, MES, MOPS, etc), with the care of using a limiting concentration in the case of the presence of chelating agents in the conjugation buffer (eg. up to 1 mM in the case of EDTA or 100 mM in the case of Tris, HEPES, MOPS, citrate, etc). In a preferred embodiment enzyme binding is carried out in 20 mM HEPES buffer pH 8.

Therefore, in an embodiment of the first aspect, the system comprising at least two substantially homogeneous and colloidal populations of MNPs is characterized in that each of the populations of MNPs are functionalized with at least one different enzyme. In another embodiment, each of the at least two populations of MNPs is functionalized with an enzyme, being the enzyme similar among the plurality of MNPs forming each of the populations, but different between each population.

The optimum temperature (OT) of an enzyme is the temperature range in which a maximal rate of reaction is achieved. Thus, in another embodiment of the first aspect of the present invention, the OT of the enzymes functionalized on each of the populations of MNPs is different among them. In another embodiment, the OT of the enzymes on each of the populations of MNPs differs among them in at least 1 Celsius degree (°C). In another embodiment, the OT of the enzymes differs in at least 1, 2, 3, 4, 5, 6, 7, 8, 9, 10, 20, 30, 40, 50, 60, 70, 80 or 90 °C among them. In a preferred embodiment, the OT of the enzymes functionalized on each of the populations of MNPs differs in at least 10 °C. A difference of at least 10 °C between the OT of two enzymes means that if, for instance, the OT of one enzyme is 70°C, then OT of the other enzyme may be equal or less than 60°C or equal or more than 80°C.

In a preferred embodiment, the enzymes attached on the surface of the MNPs are selected from the group consisting of thermophilic, hyperthermophilic, or mesophilic enzymes. By "thermophilic" enzymes is meant enzymes that work optimally between 50 and 80°C. By "hyperthermophilic" enzymes is meant enzymes that work optimally at more than 80°C. By "mesophilic" enzymes is meant enzymes that work optimally at around 37°C.

In an embodiment, the enzymes attached or functionalized on the surface of the MNPs are selected from the group consisting of oxidoreductases, transferases, hydrolases, lyases, ligases, and isomerases. In a preferred embodiment, the enzymes are selected from the group consisting of Sucrose phosphorylase from *Leuconostoc mesenteroids,* *Bacillus stearothermophilus* alcohol dehydrogenase, *Thermus thermophilus* alcohol dehydrogenase, amylase from *Bacillus licheniformis,* glucose isomerase from *Suncus murinus,* cellobiose phosphorylase from *Clostridium thermocellum,* L-aspartate oxidase from *Sulfolobus tokodaii, Bacillus megaterium* or *Chromobacterium violaceum* transaminases, *Acetobacter pasteurianus* pyruvate decarboxylase, collagenase from *Bacillus sp,* hyaluronidase from *Thermasporomyces composti, Thermus thermophilus* lipase and NADH-oxidase, horseradish peroxidase, *Thermus thermophilus,* *Saccharolobus shibatae* uracil phosphoryl transferase, or *Escherichia coli* cytosine deaminase.

In an embodiment, the enzyme attached on the surface of the MNPs of each of the populations of MNPs comprises a tag. In a preferred embodiment, the tag is Histidine x6 tag.

In an embodiment, the local thermal energy dissipated on the surface of each of the populations of MNPs upon application of an external AMF is sufficient to activate and/or inactivate each of the enzymes functionalized on said MNPs. In a preferred embodiment, the dissipated thermal energy on the surface of each of the at least two populations of MNPs is within the range of from 30-90° degrees.

It is to be understood that each of the embodiments described above for the first aspect can be combined to define the specific characteristics of the system according to the first aspect. For instance, in an embodiment, the at least two substantially homogeneous and colloidal populations of magnetic nanoparticles (MNPs) comprised in the system of the first aspect are characterized by having a particle-size distribution of less than 0.5, preferably less than 0.1, an iron oxide core, and:

i) an average particle diameter of between 8-15 nm, are coated with DMSA, functionalized with NTA and a divalent ion, preferably $Cu^{2+}$, and have a magnetic anisotropy of 34 ± 25%, preferably 27 mT;
ii) an average particle diameter of between 18-25 nm, are coated with PMAO, functionalized with NTA and a divalent ion, preferably $Cu^{2+}$, and have a magnetic anisotropy of 34 ± 25%, preferably 34 mT; or
iii) an average particle diameter of between 25-35 nm, are coated with PAA, functionalized with NTA and a divalent ion, preferably $Cu^{2+}$, and have a magnetic anisotropy of 34 ± 25%, preferably 41 mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz,
v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

With respect to the magnetic anisotropy of the system of a) and the external AMF of b) of the first aspect, the following embodiments are comprised:
It is noted that, as explained above, all of the embodiments defined for step a) can be combined with any embodiment of step b), which are described below.

By "external alternating magnetic field" (AMF) is meant Alternating magnetic fields (AMFs) that causes magnetic nanoparticles (MNPs) to dissipate heat.

In an embodiment, the external AMF comprises a parameter of magnetic flux density and a parameter of frequency. The magnetic flux density or magnetic induction, measured in tesla (T), is the number of field lines passing through a unit area of material. It is noted that terms "magnetic field", "magnetic flux" and "flux density" are often used as synonyms.

The characteristics of the system of population of MNPs as defined above in the first aspect or in any of its embodiments can be combined with any of the external AMF settings (magnetic flux density and frequency values) that are detailed below.

In an embodiment, the external AMF is characterized by a magnetic flux density applied in the ranges from 1 to 100 mT. In another embodiment, the external AMF is characterized by a magnetic flux density applied in the ranges from 5 to 90, 5 to 80, 5 to 70, 5 to 60, 5 to 50, 5 to 40, 5 to 35, 5 to 30, 5 to 25, 5 to 20, 5 to 15, 5-10 or 1-10 mT. In a further embodiment, the external AMF is characterized by a magnetic flux density applied in the ranges from 5 and 70 mT. In a preferred embodiment, the external AMF is characterized by a magnetic flux density applied in the ranges from 5-20, 10-20, 20-30, 30-40, 40-50, 50-60 or 60-70 mT.

By "frequency" is meant the number of cycles per second (from maximum positive to maximum negative and back) of the magnetic field. The more cycles that occur per second, the higher the frequency. In an embodiment, the external AMF is characterized by a frequency applied in the ranges from 1 to 1000 kHz. In another embodiment, the external AMF is characterized by a frequency applied in the ranges from 1 to 900, 1 to 800, 1 to 700, 1 to 600, 1 to 500, 1 to 400, 1 to 300, 1 to 200, 1 to 100, 1 to 750, or 1 to 50 kHz. In a preferred embodiment, the external AMF is characterized by a frequency applied in the ranges from 50 to 800 kHz. In a further preferred embodiment, the external AMF is characterized by a frequency applied in the ranges from 50 and 800 kHz. In an embodiment, an external AMF of low frequency refers to a frequency applied of less of 100 kHz. In an embodiment, an external AMF of medium frequency refers to a frequency applied that ranges from 100 to 400 kHz. In an embodiment, an external AMF of high frequency refers to a frequency applied that is higher than 400 kHz.

It is noted that, in order to define the external AMF applied, any combination of the magnetic flux density ranges or values with the frequency range or values as defined above can be combined. Thus, in an embodiment, the external AMF applied can be selected from the group consisting of i) magnetic flux density range of 40-50 mT and frequency range of 80-120 kHz; ii) magnetic flux density range of 5-20 mT, and frequency range of 300-400 kHz; iii) magnetic flux density range of 10-20 mT, and frequency range of 400-500 kHz; iv) magnetic flux density range of 50-60 mT, and frequency range of 50-100 kHz; v) magnetic flux density range of 50-60 mT, and frequency range of 50-100 kHz; vi) or magnetic flux density range of 20-30 mT, and frequency range of 600-800 kHz; vii) magnetic flux density range of 5-20 mT, and frequency range of 600-800 kHz.

Therefore, in an embodiment, one or more external AMFs are applied to the system of at least two substantially homogeneous and colloidal populations of MNPs. In a preferred embodiment, the process of the first aspect of the invention is to carry out two or more simultaneous enzymatic reactions, wherein the external AMFs applied in step b) is sufficient to produce the simultaneous activation and/or inactivation of the enzymes functionalized on the surface of each of the populations of MNPs.

In a preferred embodiment, the process of the first aspect of the invention is to carry out two or more sequential enzymatic reactions, wherein the one or more applied external AMFs of step b) are applied sequentially so as to produce the sequential activation and/or inactivation of the enzymes functionalized on each of the surfaces of each of the populations of MNPs.

It is key in the present invention to define the conditions of applied field ($H_{max}$) and magnetic field anisotropy ($H_A$) that maximize the heat dissipation. The best strategy to maximize the SAR is to maximize the anisotropy of the system up to the point in which the applied field is able to perform major loops.

In an embodiment, the value of the external field in relation with $H_A$ is considered. In another embodiment, the ratio between $H_{MAX}$ and $H_A$ is taken as criteria to predict the heating power of MNPs at a certain applied field. In a further embodiment, for a randomly distributed non-interacting system the hysteresis losses will be negligible for $H_{max}/H_A < 0.5$ [Munoz-Menendez et al. 2017].

**For instance, Figure 10A** shows how the area of the major loops increases by increasing the K of the nanoparticles. The red cycle, which corresponds to MNPs with high K, is wider and have larger area whereas the area is reduced in the narrow blue cycle that corresponds to MNPs with small K. **Figure 10B** analyzes the dependence between the normalized hysteresis loses (Loop areas=A/2KV) and the $H_{MAX}$ (applied field). Therefore, in an embodiment, the systems start to dissipate heat when the $H_{max}/H_A \approx 0.5$ and reaches the maximum when $H_{max}/H_A \approx 0.75$. As a general rule, it can be observed that in the case of interacting nanoparticles the maximum of hysteresis loses is achieved for $H_{max}/H_A = 1.1$ or greater, and over this limit the energy introduced by higher $H_{MAX}$ is not absorbed by the MNPs.

Thus, in the context of the present invention, the following can be considered:

- Negligible dipolar interactions between magnetic nanoparticles (assured by the colloidal stability of the suspensions, only achieved after proper coating).
- All the heat dissipation mechanisms are included in the magnetic loops (no heating from the hyperthermia equipment).
- Anisotropy of the nanoparticles are defined by the contribution of shape and crystal (surface anisotropy is considered negligible for particle sizes over 10 nm where surface represents less than 25% of the volume).

In an embodiment, the value ratio $H_{max}/H_A$ comprises any value in between 0.4 and 1.5. In an embodiment, the ratio between the magnetic flux density and the magnetic anisotropy is greater than 0.4. In a preferred embodiment, the ratio between the magnetic flux density and the magnetic anisotropy is greater than 0.5. In another embodiment, the ratio between the magnetic flux density and the magnetic anisotropy is between 0.4 and 1.2. In a preferred embodiment, the ratio between the magnetic flux density and the magnetic anisotropy is between 0.5 and 1.1.

In another embodiment, the magnetic anisotropy of each of the populations of MNPs is within the range of 34 mT $\pm$ 50%, and the external AMF is characterized by a frequency range between 50 and 800 kHz and a magnetic flux density of between 5 and 70 mT, wherein, preferably, the ratio between the magnetic flux density and the magnetic anisotropy is greater than 0.4, preferably greater than 0.5. In a preferred embodiment, the magnetic anisotropy of each of the populations of MNPs is within the range of 34 mT $\pm$ 50%, and the external AMF is characterized by a frequency range between 50 and 800 kHz and a magnetic flux density between 5 and 70 mT, and the ratio between the magnetic flux density and the magnetic anisotropy is between 0.4 and 1.2. In an even more preferred embodiment, the magnetic anisotropy of each of the populations of MNPs is within the range of 34 mT $\pm$ 50%, and the external AMF is characterized by a frequency range between 50 and 800 kHz and a magnetic flux density of between 5 and 70 mT, and the ratio between the magnetic flux density and the magnetic anisotropy is between 0.5 and 1.1.

In a preferred embodiment, the at least two substantially homogeneous and colloidal populations of magnetic nanoparticles (MNPs) comprised in the system of the first aspect are characterized by having a particle-size distribution of less than 0.5, preferably less than 0.1, an iron oxide core, and:

i) an average particle diameter of between 8-15 nm, are coated with DMSA, functionalized with NTA and a divalent ion, preferably $Cu^{2+}$, and have a magnetic anisotropy of 34 $\pm$ 25%, preferably 27 mT;
ii) an average particle diameter of between 18-25 nm, are coated with PMAO, functionalized with NTA and a divalent

ion, preferably $Cu^{2+}$, and have a magnetic anisotropy of $34 \pm 25\%$, preferably 34 mT; or
iii) an average particle diameter of between 25-35 nm, are coated with PAA, functionalized with NTA and a divalent ion, preferably $Cu^{2+}$, and have a magnetic anisotropy of $34 \pm 25\%$, preferably 41 mT;

and wherein the one or more AMF applied in b) are selected from the group consisting of:

i) magnetic flux density range of 40-50 mT and frequency range of 80-120 kHz,
ii) magnetic flux density range of 5-20 mT, and frequency range of 300-400 kHz,
iii) magnetic flux density range of 10-20 mT, and frequency range of 400-500 kHz,
iv) magnetic flux density range of 50-60 mT, and frequency range of 50-100 kHz,
v) magnetic flux density range of 50-60 mT, and frequency range of 50-100 kHz, or
vi) magnetic flux density range of 20-30 mT, and frequency range of 600-800 kHz.
vii) magnetic flux density range of 5-20 mT, and frequency range of 600-800 kHz.

With respect to the reaction media of the first aspect, the following embodiments are comprised:
It is noted that, as previously explained, all of the embodiments defined for step a) and step b) can be combined with any embodiment relating to the reaction media, which are explained below.
In an embodiment, the reaction media according to the first aspect of the present invention or any of its embodiments is kept during step b) at a significantly lower temperature than the lowest optimum temperature of the enzymes functionalized on the surface of each of the populations of MNPs. This can be achieved for example by controlling the concentration of MNPs in the reactor (Figure 12), such as by maintaining a concentration of MNPs of between 0.05-0.1 mg/mL, since this concentration range does not affect the temperature of the reaction media as to negatively affect the overall process provided in the present invention.
By "significantly lower" is meant at least 100, 90, 80 ,70, 60, 50, 40, 30, 20, 10, 5°C less than the lowest optimum temperature of the enzymes functionalized on the surface of each of the populations of MNPs.
In an embodiment, the reaction media is an aqueous solution. The optimum temperature (OT) of the medium is the one that does not interfere with the enzymatic reaction carried out by the enzymes functionalized on the surface of the MNPs of the at least two substantially different populations of MNPs. Thus, in an embodiment, the temperature of the reaction media is set forth not to interfere with the enzymatic reactions that will be carried out and is kept at this temperature or at another temperature as long as it never interferes with the enzymatic reactions. By "not to interfere with the enzymatic reaction" is meant that the temperature of the media does not causes undesired activation or deactivation of the enzymes functionalized on the surface of each of the populations of MNPs. In order to achieve that, the OT of the enzymes attached to the MNPs needs to be considered, in particular the lowest OT among them. For instance, if the lowest OT of the enzymes present in the system is 40°C, then the reaction media needs to be significantly lower than this temperature. In an embodiment, the temperature of the reaction media is and is kept at a temperature of at least 1, 2, 3, 4, 5, 10, 15, 20, 25, 30, 35, 40, 45, 50, 55, 60, 65, 70, 75, 80, 85, or 90 °C lower than the lowest OT of the enzymes functionalized on the surface of each of the populations of MNPs. Preferably, the temperature of the reaction media is between 0 and 40°C, more preferably between 4 and 37°C.
It is noted that, depending on the heat dissipated by the MNPs, the reaction media might change its temperature. In an embodiment, the temperature of the reaction media increases about $\pm$ 1°C in 15 minutes when the reaction media comprises a concentration of MNPs equal or greater than 0.007 mg of MNPs/mL (0.005 Fe mg /mL). In an embodiment, the temperature of the reaction media increases about $\pm$ 2°C in 15 minutes when the reaction media comprises a concentration of MNPs equal or greater of 0.07 mg of MNPs/mL (0.05 Fe mg /mL). In an embodiment, the temperature of the reaction media increases about $\pm$ 5°C in 15 minutes when the reaction media comprises a concentration of MNPs equal or greater of 0.35 mg of MNPs/mL (0.25 Fe mg /mL).
In a further embodiment, when the MNPs are comprised of Fe compounds, the temperature of the reaction media increases about $\pm$ 1°C in 15 minutes when the reaction media comprises a concentration of MNPs equal or greater of 0.005 mg of Fe/ml. In a further embodiment, when the MNPs are comprised of Fe, the temperature of the reaction media increases about $\pm$ 2°C in 15 minutes when the reaction media comprises a concentration of MNPs equal or greater of 0.05 mg of Fe/ml. In a further embodiment, when the MNPs are comprised of Fe, the temperature of the reaction media increases about $\pm$ 5°C in 15 minutes when the reaction media comprises a concentration of MNPs equal or greater of 0.25 mg of Fe/mL.
In an embodiment, the reaction media according to the first aspect of the present invention or any of its embodiments further comprises a suitable buffer solution. In an embodiment, the buffer solution is selected from the group consisting of Na or K-phosphate, phosphate citrate, HEPES, MES, MOPS, sodium acetate, etc. Preferably, the buffer solution comprised in the reaction media is HEPES.
In an embodiment, the reaction media is adjusted to the conditions required to carry out the enzymatic reaction. In another embodiment, the reaction media according to the first aspect of the present invention or any of its embodiments is at a suitable pH according to the enzymatic process to be carried out. In a preferred embodiment, the pH of the reaction media ranges from 6 to 8. More preferably, the pH of the reaction media is 7.

In another embodiment, the process according to the first aspect of the present invention or any of its embodiments is carried out during sufficient time to perform the enzymatic reaction. In an embodiment, the reaction time is about 1, 5, 10, 15, 20, 25, 30, 35, 40, 45, 50, or 60 minutes. In a further embodiment, the process is carried out during at least about 1, 2, 3, 4, 5, 6, 7, 8, 9, 10 or more than 10 hours. Further, in another embodiment, several cycles with similar or different times can be performed. Of note, the term "about" can refer the indicated value $\pm 10\%$.

In an embodiment, the reaction media further comprises other molecules such as cofactors, enzymes, ions, organic molecules or inorganic molecules.

In a **second aspect,** the present invention relates to a system comprising at least two substantially homogeneous and colloidal stable populations of magnetic nanoparticles (MNPs), characterized in that each of the populations of MNPs are functionalized with at least one different enzyme, and characterized in that each of the populations of MNPs have a different magnetic anisotropy capable of dissipating local thermal energy on the surface of each of the population of MNPs upon application of an external alternating magnetic field (AMF) sufficient to activate and/or inactivate each of the enzymes functionalized on said MNPs.

In an embodiment of the second aspect, the system is characterised as defined in step a) and b) of the first aspect or in any of its embodiments. Thus, the embodiments related to the average particle diameter, particle-size distribution, core, shell, magnetic anisotropy, coating, functionalization, chelating agent, divalent metal, optimum temperatures, enzymes, AMF settings applied to them, the dissipated thermal energy, and any combination thereof as defined above are also applied here and must be considered as embodiments of this aspect.

In an embodiment of the second aspect, the system further comprises a device suitable for generating the external AMF as defined in the first aspect or in any of its embodiments. In a preferred embodiment, the device suitable for generating the external AMF comprises suitable AMF-generating electromagnets and their driving circuits to produce the external AMF or a coil array. The device may include electromagnets employing soft ferromagnetic cores that operate offering a high permeability path for magnetic flux magnetized by current-carrying wire with a comparatively small air gap that dominates the overall reluctance or a conductive solenoid operating at high frequencies AC currents that generates a highly homogeneous field along its central axis. Examples of suitable devices are Five Celes ICMM and nB nanoScale Biomagnetics DM100 series.

In a **third aspect,** the present invention relates to the *in vitro* use of the system according to the second aspect or any of its embodiment to carry out the enzymatic process according to the first aspect or any of its embodiments.

In an embodiment, the *in vitro* use of the system according to the third aspect includes uses in industrial processes, batch or continuous flow production on traditional industrial enzyme reactors, continuous flow production on microfluidic reactors and lab-on-a-chip systems of analysis. Particularly, in an embodiment, microfluidic chips for use in assays for carrying out enzymatic processes are also included. The term "microfluidic chip or device" refers to a set of micro-channels etched or molded into a material (such as glass, silicon, a thermoplastic material or a polymer such as PDMS, for PolyDimethylSiloxane). The micro-channels forming the microfluidic chip are connected together in order to achieve the desired features (mix, pump, sort, control bio-chemical environment). This network of micro-channels trapped into the microfluidic chip is connected to the outside by inputs and outputs pierced through the chip, as an interface between the macro- and micro-world. It is through these holes that the liquids (or gases) are injected and removed from the microfluidic chip (through tubing, syringe adapters or even simple holes in the chip) with external active systems (pressure controller, push-syringe or peristatic pump) or passive ways (e.g. hydrostatic pressure).

The *in vitro* use of a microfluidic chip or kit or device comprises a support or substrate, wherein said support or substrate comprises at least one channel in the substrate, the channel comprising an inlet, an outlet, and a flow-path connecting the inlet and outlet, wherein the inlet and outlet together define a midplane; and a portion of the flowpath travels transversely across the midplane, wherein the portion of the flowpath that travels transversely across the midplane includes a recognition site or sensing area for detecting, for instance, a target analyte or for detecting an analyte as a result of the heat generated by the magnetic nanoparticles. The materials mentioned before (glass, silicon, a thermoplastic material or a polymer such as PDMS) are perfectly compatible with the use of high frequency AMF. Besides, the use of microfluidics simplifies the application of AMF reducing the active volume in which the homogeneous AMF needs to be induced.

A detailed description of the invention is herein provided by means of the following examples, without the intention of limiting its scope of protection.

## EXAMPLES

## EXAMPLE 1: Materials and Methods

*1- Iron oxide MNP preparation.*

Three different iron oxide MNPs were produced by three different methods: coprecipitation in aqueous media, thermal decomposition in organic media and oxidative precipitation in aqueous media. **Figure 2** shows the TEM images of the monodisperse populations of MNPs obtained by coprecipitation (CP), thermal decomposition (TD) and oxidative precipitation (OP).

∘ *1.A Preparation of Iron Oxide MNP by coprecipitation in aqueous media.*

System A MNPs were synthesized by Massart coprecipitation protocol. In short, 445 ml of a mixture of $FeCl_3 \cdot 6H_2O$ (0.09 mol) and $FeCl_2 \cdot 4H_2O$ (0.054 mol) were added to 75 ml of $NH_4OH$ (25%). The ferrous salt solution was slowly poured in the base at room temperature with vigorous stirring. To enlarge the size of MNPs prepared by this method, the mixture was heated up to 90°C for 1 h and left to cool down to room temperature. The final product (aprox. 10 g) was washed three times with distilled water by magnetic decantation.

To complete the oxidation of MNPs prepared by coprecipitation and oxidative precipitation and obtain a single phase of maghemite ($\gamma$-$Fe_2O_3$), the samples were treated with $HNO_3$ and $Fe(NO_3)_3$. This procedure, referred as acid treatment, is based on an oxidation and dispersion process previously reported. The MNPs were treated with 300 mL of $HNO_3$ (2 M) for 15 min. Then, nitric acid was removed by magnetic decantation, and 75 mL of $Fe(NO_3)_3$ (1 M) and 130 mL of water were added. The mixture was heated up to 90°C and stirred for 30 min. The particles were then cooled to room temperature, and by magnetic decantation, the supernatant was substituted by 300 mL of $HNO_3$ (2 M) for 15 min. Finally, they were washed three times with acetone and redispersed in water. A rotary evaporator was used to remove any acetone waste and concentrate the sample.

MNPs prepared by coprecipitation were coated with dimercaptosuccinic acid (DMSA) using an aqueous approach. 5 mg (0.027 mol) of DMSA were added dissolved in 5 mL of deionized water and mixed at 50°C under vigorous stirring. The DMSA solution was added to a suspension of 5 mL of MNPs (8.6 g $Fe_2O_3$/L) at pH 3 and sonicated for 1 minute. The pH of the mixture was raised to 11 and sonicated for 20 min. After this time, the sample was dialyzed for 48 hours.

∘ *1.B Preparation of MNPs by thermal decomposition in organic media*

System B MNPs were obtained by decomposition of Fe(III) acetylacetonate (100 mM) in dibenzil eter (1 L) in the presence of oleic acid (300 mM) and 1,2 dodecanodiol (200 mM) under nitrogen flow (9 L/min) and 100 rpm stirring. The mixture was heated up to 200°C at 3°C/min and kept for 2 h. Then, the temperature was increased up to the boiling point (295°C) at 6°C/min and kept for another hour. The reaction was stopped by removing the mantle and leaving the mixture to cool down. The washing process consists on precipitating the nanoparticles with a mixture of hexane/ethanol (1:3) vol (final volume 2500 mL) and discarding the supernatant containing by-products, surfactants and rest of solvents after two days of magnetic separation. Three washing steps with 150 cc of toluene/ethanol (1:2) vol leaded to the magnetite/maghemite final product (6.2 g) which was redispersed in 170mL of a solution of oleic acid in toluene 13%wt using an ultrasound bath (15min).

In order to transfer this MNPs to water, their coating with poly(maleic anhydride-alt-1-octadecene (PMAO). Briefly, 224 mg of PMAO (MW: 30000 and 50000 Da) were added to a 500 mL flask containing 198 mL of chloroform. After dissolving the polymer under magnetic stirring, 20 mg of the hydrophobic nanoparticles (previously washed three times with ethanol and resuspended in 2 mL of chloroform) were added drop and wise in an ultrasonic bath and the mixture was kept in the bath for 15 minutes at room temperature. Then the most part of the chloroform was removed under vacuum leaving a volume of 5 - 10 mL solution. 10 mL of Milli-Q water and 10 mL of 0.1 M NaOH were added at once (foaming occurs due to the hydrolysis of the maleic anhydride groups) and the solvent was removed at 70 ° C increasing the vacuum progressively until the complete disappearance of foam. The solution was then filtered using a 0.2 $\mu$m syringe filter. Finally, four ultracentrifugation steps were carried out during 2 hours at 24000 rpm at 15°C to eliminate unbound polymer.

∘ *1.C Preparation of MNPs by oxidative precipitation in aqueous media*

System C of large MNPs with rhombohedral shape were prepared by a direct method in aqueous medium called oxidative precipitation. Briefly, a 1M solution of $FeSO_4$ was prepared dissolving 13.90 g $FeSO_4 \cdot 7H_2O$ in 50 mL of $H_2SO_4$ 0.01M. The ferrous solution was quickly added to a basic solution prepared with 4.25 g of $NaNO_3$ and 4.22 g of NaOH in a mixture of 137 mL of water and 63 mL of ethanol 96% vol. The suspension of green rust obtained was stirred for 15 min and poured in a jacketed flask previously thermalized to 90 ᴼC with an oil thermostatic bath. The MNPs were left to grow at 90ᴼC for 6 hours. The whole process was performed in a glove box under nitrogen atmosphere to avoid the formation of secondary Fe oxide phases. The final precipitate was washed with distilled water by magnetic decantation obtaining 3.8 g of magnetite/maghemite nanoparticles.

MNPs prepared by this route were subjected to an acid treatment already described for the coprecipitation particles and then, coated with PAA (1.2 kDa)[Costo et al. 2019]

## 2 - Functionalization of MNPs with NTA and a metal (Me$^{2+}$= Cu$^{2+}$, Co$^{2+}$ and Ni$^{2+}$) (Materials and Methods)

NTA-Me$^{+2}$ moieties were introduced on MNPs with coatings containing carboxyl moieties in order to specifically bind his-tagged recombinant proteins by metal affinity binding. The following coating were performed:

○ NTA-Me$^{+2}$ complex preparation

For this step, a modified nitrile acetic acid (NTA) molecule which contains an extra tail ending in a primary amine group (NH$_2$) was used. The preparation of NTA-Me$^{2+}$ was performed by preparing a suspension containing 45 mM of N$_\alpha$-Bis(carboxymethyl)-L -lysine hydrate (NTA; Sigma Aldrich, USA) and 50 mM of CuSO$_4$ or NiCl$_2$ or CoCl$_2$. The pH of the solution was adjusted to 10.5 using 2 M sodium hydroxide (Sigma Aldrich, USA). The solution obtained was centrifuged at 4000g for 10 minutes to eliminate the unreacted salt. The supernatant was collected and its pH was adjusted between 8.5 and 8.9.

○ NTA-Me$^{+2}$ functionalization of MNPs

**Systems A and C:** To functionalize the MNPs, a solution containing 2 mM 1-3-dimethylaminopropyl-3-ethylcarbodiimide (EDC; Sigma Aldrich, USA) and 0.8 or 8 mM N-hydroxysulfosuccinimide (s-NHS and Sigma Aldrich, USA) was prepared in 10 mM MES pH 7.0 or 20 mM HEPES pH 8.0 and incubate at room temperature for 10 min, after the volume of particles corresponding to 1 mg$_{Fe}$ of MNPs added and the volume adjusted to 1 mL. To this suspension 500 μL of 45 mM NTA-Me$^{2+}$ was added. The obtained suspension was incubated at room temperature for 2 hours. Then a solution of 520 nM α-Methoxy and ω-amino poly(ethylene glycol) of 5000 Da (PEG-5000; RappPolymer, Germany) was poured into the suspension to block the unreacted carboxylic groups and to increase the colloidal stability of the particles. After 45 minutes of stirring at room temperature, the particles were separated with a magnet or by centrifugation at 4000 g for 5 minutes. The obtained conjugates were suspended in fresh 10 mM MES buffer pH 7.0 or 20 mM HEPES buffer pH 8.0 and stored at 4°C. The supernatant of the reaction was collected and stored at 4°C for further analysis.

**System B**: NTA-Me$^{2+}$ complex was incubated in 500 μL of 50 mM Borate Buffer pH 8.0SSB for 5 minutes. Then, were centrifuged at 4500 for 5 minutes. The supernatant was collected and added to a solution containing 23 μM α-Methoxy-ω-amino poly(ethylene glycol) PEG 750 Da (PEG-750, RappPolymer, Germany). Then PMAO functionalized MNPs (1 mg of Fe) were added to the solution above. Then 40 μmol of EDC was suspended in 20 μL and added to the suspension and stir at room temperature and protected from light. After 30 minutes, the same amount of EDC was added to the MNPs suspension. The mixture was stirred for three hours protected from light at room temperature. To eliminate the excess of reagents several washing steps with Milli-Q water by centrifugation (5000 rpm for 15 min, 2 times) were carried out using 4 mL cellulose membrane centrifugal filters (Amicon, MilliPore, 100 kDa). The particles were recovered from the filters using Milli-Q water and stored at 4 °C.

## 3 - Structural characterization.

The size and morphology of the particles were determined by transmission electron microscopy (TEM, JEOL JEM 1010) operating at 100.0 keV. The size distribution in the samples was determined measuring more than 150 MNPs with ImageJ digital software and adjusting the result with a log and normal fitting. Crystalline structure was determined by X-ray diffraction using Bruker D8 Advance diffractometer with a Cu Kα radiation lamp in Bragg and Brentano configuration. The crystal size was obtained from the width of (311) spinel peak using Scherrer equation. Hydrodynamic size and Z-potential were determined with a Malvern Instrument Zetasizer Nano SZ (Malvern, UK) operating at 633 nm. Fe concentration in the colloids were determined by inductively coupled plasma optical emission spectroscopy (ICP and OES, ICP PERKIN ELMER mod. OPTIMA 2100 DV), digesting a controlled volume of the sample. Fourier and Transformed spectra were measured in a Bruker IFS 66VS with a spectral range of 400-4000 cm$^{-1}$. The samples were dried and mixed with KBr to create a compacted pellet. Thermogravimetric curves (ATD/DSC/TG, Q600 TA Instruments) were obtained also from dried samples heating from room temperature to 900°C in air at a heating rate of 10°C/min.

## 4 - Magnetic and calorimetric characterization.

Magnetic characterization was carried out in a Vibrating Sample Magnetomerter (MagLabVSM, Oxford Instru-ment). 50 μL of MNPs were dropped in a cotton pellet and dried in an oven at 50°C overnight. The dried cotton was compacted in a standard gelatine capsule. The magnetization values were normalized to the amount of Fe in the volume dried determined by ICP and OES.

Calorimetric measurements were carried out in a AMF inductor (Fives Celes, MP 6 kW) with an optic fibre thermal probe (OSENSA Innovations) introduced in the sample holder. Non-adiabatic heating curves were acquired by irradiating 1 mL of MNPs colloid with and Fe concentration of 1 g L$^{-1}$ with two AMF conditions: 100 kHz and 40 kA m$^{-1}$ and 300 kHz and 13 kA m$^{-1}$.

The heating curve slope was determined by fitting the 20 initial seconds with a linear fit. Heating power of MNPs was quantified using the specific absorption rate (SAR) obtained from equation 1:

$$SAR = \frac{C_V}{[Fe]} \Delta T \big/ \Delta t \quad (1)$$

Where $C_V$ is the water specific heat, [Fe] is the Fe concentration in the colloid and $\Delta T/\Delta t$ is the slope determined from heating curves. Two different devices have been used to study the effect of AMF:

1. NB D5 series: frequency range 94 and 763 kHz; field range 5 and 50 mT. Equipped with a high vacuum pump and chiller set to 17°C.
2. Fives Celes: frequency range 90 and 300 kHz; Field range 10 and 60 mT. Equipped with a chiller set to 13°C.

*5 - Metal affinity conjugation of GFP and mC proteins to MNPs.*

The Green Fluorescent Protein (GFP) and m-Cherry (mC) conjugation was carried on by incubating 50 $\mu g_{protein}$ with 100 $\mu g_{Fe}$. in 20 mM HEPES buffer pH 8. The reaction was maintained under a continuous stirring at room temperature for 2 hours protected from light. The particles were then separated with a magnet, dispersed in fresh buffer and stored at 4°C. The supernatant was collected for further analysis.

*6 - Protein concentration determination*

The protein concentration was determined by Bradford assay using Bovine Serum Albumin (BSA, Sigma Aldrich, USA) as standard. Briefly, 150 $\mu$L of each standard or unknown sample were pipetted into the appropriate microplate wells. Then, 150 $\mu$L of the Coomassie Plus Reagent (Thermofisher Scientific, USA) were added to each well and mixed for 30 seconds. The plate was incubated for 10 minutes at room temperature, then the absorbance at 595nm was measured using a Thermofisher scientific plate reader.

*7 - Protein yield*

The amount of protein bound to the carrier is expressed as the difference between the amount of protein offered in reaction and the amount of protein unbound in the solution (Equation 2).

$$P_{immob} = P_{offered} - P_{sup} \quad\quad\quad (2)$$

Where: $P_{immob}$ represents the amount of protein bound to the MNPs, $P_{offered}$ is the amount of protein offered in reaction and $P_{sup}$ is amount of unbound protein, found in the in the supernatant and the washing steps.

The immobilization yield (Y) was calculated as the ratio between the amount of protein immobilized on to the carrier and the amount of protein offered to the carrier (Equation 3).

$$Y(\%) = \frac{P_{immob}}{P_{offered}} \times 100 \quad\quad\quad (3)$$

*8 - Local heating determination by a fluorescent probe.*

∘ *Global heating effect on GFP and mC fluorescence.*

The samples were diluted to contain 10 $\mu$g/mL of GFP/mC and the dependence of its fluorescence with temperature was studied by heating the GFP/mC solution globally using a thermoblock. Each protein aliquot was incubated for 5 minutes at different temperature in a thermomixer under shaking at 350 rpm. After this 0.5 M imidazole (Fluka, USA) was added to the sample to elute the protein from the particles. The samples were incubated 30 minutes at room temperature under stirring, then the particles were separated from the supernatant by centrifuging 5 minutes at 12000 rpm. The supernatant excitation and emission spectra were collected (excitation: 488 nm; emission:510 nm for GFP and excitation: 588 nm; emission: 610 nm for mC) using a spectrofluorometer (PerkinElmer, USA). Soluble GFP and mC were used as controls at the same concentration used for conjugated ones.

∘ *Effect of the application of an alternating magnetic field to the conjugated GFP and mC fluorescence.*

Samples were diluted to contain 10 μg/mL of GFP/mC and placed into an AMF inductor. The AMF at different settings (frequency and field intensity combinations) was applied for 5 minutes. After the treatment, 0.5 M imidazole (Fluka, USA) was added to the sample to elute the protein from the particles. The samples were incubated 30 minutes at room temperature under stirring, then the particles were separated from the supernatant by centrifuging 5 minutes at 12000 rpm. The sample and excitation and emission spectra were collected (excitation: 488 nm; emission:510 nm for GFP and excitation: 588 nm; emission: 610 nm for mC) using a spectrofluorometer (PerkinElmer, USA). The fluorescence collected with the samples stored at Room Temperature was used as a control.

9 - Reaction media temperature analysis.

Once determined the optimum AMFs conditions for thermal dissipation in the three systems, it was evaluated the heating power of each system at optimum conditions when the concentration is diluted to operative concentrations in enzymatic reactions. The samples were prepared by diluting concentrated dispersions of MNPs in distillate water. The temperature increase was measured as indicated in section 4, applying the AMF for 15 min. Figure 12 show the heating curves of the colloids for Fe concentrations of 1, 0.25, 0.05, 0.01, 0.005 mg of Fe/ mL.

**EXAMPLE 2: Toolbox of MNPs**

The three chosen populations of MNPs are iron oxide MNPs of different sizes and shapes, like those presented in Figure 2. System A consists on a MNPs population of iron oxide spheroidal nanoparticles with an average diameter of 8.3 nm prepared by coprecipitation (CP) and coated with a dimercaptosuccinic acid (DMSA). System B consists on a MNPs population of iron oxide spheroidal nanoparticles with an average diameter of 22 nm prepared by thermal decomposition (TD) and coated with oleic acid and poly(maleic anhydride-alt-1-octadecene (PMAO). System C consists on rhombohedral iron oxide nanoparticles with an average diagonal size of 32 nm prepared by oxidative precipitation (OP) and coated with polyacrylic acid (PAA). The CP synthesis generates spheroidal MNPs with an average size of 8.3 nm ($\sigma$=0.24). The MNPs prepared by TD present larger average diameter ($D_{TEM}$= 20.2 nm, $\sigma$=0.23) and a quasi-spheroidal shape. The largest MNPs were obtained by OP. They present a rhombohedral geometry with an average size of 33.2 nm ($\sigma$=0.23).

**EXAMPLE 3: Simultaneous and sequential thermal energy dissipation. Proof of concept using systems A, B and C.**

Regarding their functionalization, the three systems were coated with shells containing carboxylic groups in order to improve their colloidal stability by increasing their surface charge and to introduce functional groups where the thermosensitive fluorescent probe can be anchored. Systems A (8 nm) was coated with DMSA. System B (22 nm) was coated with DMSA and PMAO. For System C, the largest nanoparticles, it was necessary to coat the surface with a charged polymer (PAA) that combines electrostatic and steric repulsion to ensure its colloidal stability in biological media. **Figure 2e** shows the hydrodynamic size of the three systems after surface coating. The larger size observed for aqueous synthesis methods (CP and OP) suggests the formation of primary aggregates made of few MNP during the coating. In the case of TD, the coating with PMAO polymer on top of the oleic acid generates single MNPs with low hydrodynamic size and high stability.

As a proof of concept, systems A, B and C were chosen for the simultaneous and sequential heat activation upon application of specific AMF conditions. As shown in **Figure 3a and b,** the three systems dissipate different thermal energy (SAR) during application of low frequencies AMF. At low frequencies, the SAR scales with the volume of the MNPs. Further, **Figure 3c** shows that systems A and B present different optimum AMF conditions for the production of heat (maximum SAR). This demonstrated that the controlled thermal energy dissipation on the surface of the MNPs of the present invention is achievable combining well-differentiated populations of MNPs in a single pot system.

**EXAMPLE 4: Detailed characterization of the coated MNPs.**

The order to further study the MNPs generated and the effect of their functionalization, several physical and chemical analyses were performed: In **Figure 4a** the FT-IR spectra of system A with DMSA shows two broad peaks at 1593

and 1376 cm$^{-1}$ correspondent to asymmetric and symmetric stretch of COO$^-$ groups of DMSA respectively. System B coated with PMAO presents a combination of a wide peak at 1400 cm$^{-1}$ created by the bending modes of CH$_2$ of oleic acid chains with the 1551 and 1695 cm$^{-1}$ sharp peaks generated by the carbonyl stretching modes. Similarly, for System C, the PAA coating shows two pairs of resolved peaks at 1638/1568 cm$^{-1}$ and 1544/1405 cm$^{-1}$ that suggest the presence of both protonated and deprotonated carboxylic groups in the polymer. The broad band at 600 cm$^{-1}$ is generated by the Fe-O groups of the magnetite/maghemite in all the cases. TG curves in **Figure 4b** show different trends for MNPs coated with DMSA, PMAO and PAA. The formers present two drops of mass at 300°C and 600°C, while the later shows a single step at 280°C. The organic content resulted maximum for System B (26.1 %) since it is coated with a thick polymeric layer whereas DMSA coating in system A (11.3 %) resulted lower despite its higher relative surface due to the small size of this coating. In system C, the small mass loss observed in the TG curve (3.4%) suggest a thin coating of PAA on the surface of MNPs.

**Figure 4c** shows Z-potential measurements that are consistent with the presence of charged carboxylic groups in the surface of the MNPs. The three samples present negative Z-potentials below -25 mV at neutral pH. The high surface charge observed for PAA coating (-40 mV), with just 3.4% of organic mass, points out the efficiency of the coating method employed (high pressure) to decorate the surface of large MNPs with multiple functional groups.

The hysteresis loops presented in **Figure 5** show the magnetic response observed in each sample under quasistatic conditions. All of them present a maximum magnetization around $105 \pm 2$ kA m$^2$ kg$_{Fe}$$^{-1}$, which is consistent with maghemite saturation magnetization. The smallest nanoparticles (system A) are closer to the superparamagnetic regime and consequently present a small coercivity ($H_C$=2 kA m$^{-1}$). In the case of 22 nm System B multicore nanoparticles, the small size of the crystals implies a similar $H_C$ to System A sample. However, the collective magnetic behaviour of the nanocrystals inside arises significantly the susceptibility of the cycles. On the opposite extreme, the hysteresis loop of System C sample presents larger coercivity ($H_C$= 3 kA m$^{-1}$) and smaller susceptibility.

### EXAMPLE 5: Systems A, B and C functionalized with NTA-Cu$^{+2}$ and a fluorescent molecule protein.

Next, the three systems described and characterized above were coated with NTA-Cu$^{+2}$ as a vector molecule for the attachment of the GFP or mC. As stated above, the GFP and mC molecules used herein are both recombinant protein modified with a 6 x histidine tail (x6Hys-tag) so that they can be immobilized on the surface of the MNPs site-specifically through this tag. As shown in **Figure 6a,** MNPs were functionalized without generating a significant aggregation of them.

Further, **Figure 6b** shows a reduction of the negative charge in functionalized samples, confirming their functionalization. Next, the heating capacity of the MNPs was analysed and a reduction of SAR was observed in all functionalized samples. As shown in **Figure 6c,** although the warming capacity slightly decreased after functionalization, especially after NTA functionalization, it is observed that all the MNPs still dissipate thermal energy upon application of AMF, indicating that they maintain their heating capabilities regardless their functionalization.

Next, the immobilization yield was studied in systems A, B and C functionalized with GFP and mC.

**Table 1** below shows the immobilization yields by increasing the amount of protein provided during the conjugation reaction. Immobilization carried out in 10 mM HEPES pH 8.0. Measurements were made in duplicate. The deviation is less than 10%.

**Table 1:** Immobilization yields of the different MNPs.

| | Immobilization Yield (%) | | | | | |
|---|---|---|---|---|---|---|
| Protein offered / 0.1 mg Fe | 10 $\mu$g | | 25 $\mu$g | | 50 $\mu$g | |
| Protein | GFP | mC | GFP | mC | GFP | mC |
| System A-NTA-Cu$^{2+}$ | 95 | 96 | 95.6 | 96 | 68 | 70 |
| System B-NTA-Cu$^{2+}$ | 98 | 97 | 95.6 | 96 | 64 | 66 |
| System C-NTA-Cu$^{2+}$ | 90 | 89 | 96 | 97 | 62 | 61 |

By using imidazole, it was possible to determine the specificity and reversibility of the binding of GFP or mC to the MNPs. When imidazole competes with the histidine tail of both recombinant fluorescent proteins for its binding to immobilized chelators, thus if immobilization is through the histidine tail, no binding should be observed if it is done in the presence of imidazole. This was observed with all MNPs and both recombinant proteins, which indicates that the binding is oriented through the His-tag in all cases. On the other hand, if the protein is bound through the His-tag, the binding should be reversible, and therefore 100% of the bound protein should be eluted when the MNPs-GFP or -mC complexes are incubated with imidazole. As can be seen in **Table 2**, it was confirmed that the binding of GFP to all MNPs, in addition to being oriented, is reversible. Similar results were obtained in the case of MNPs-mC complexes.

**Table 2:** Reversibility of the binding of his-tagged proteins to MNPs, when analysing the eluted protein after incubating the GFP-MNPs derivatives for half an hour with 0.5 M Imidazole in 10 mM HEPES pH 8.0 at room temperature.

| | Protein bound ($\mu$g/0.1 mg Fe) | | Protein eluted ($\mu$g) | | Elution yield (%) | |
|---|---|---|---|---|---|---|
| Protein | GFP | mC | GFP | mC | GFP | mC |
| System A | 34 | 35 | 24 | 27 | 71 | 77 |
| System B | 32 | 33 | 31 | 29 | 97 | 88 |
| System C | 31 | 31 | 30 | 29 | 97 | 94 |

The binding yields of GFP to MNPs functionalized with other divalent metals ($Co^{+2}$ and $Ni^{+2}$) was also studied and it was found that it is similar to that obtained with $Cu^{+2}$ (**Figure 7**). Similar results were also observed regarding the specificity and reversibility of the binding with these divalent metals.

**EXAMPLE 6: GFP and m-Cherry Protein (mC) as independent molecular thermometers.**

The main purpose of the coating of MNPs with GFP and mC proteins is the study of the local temperatures induced by diluted colloids of MNPs when exposed to AMF. The tertiary structure of these proteins is affected by the temperature of the media, and this its intrinsic fluorescence.

As shown in **Figure 8c,** the fluorescence intensity of GFP (black) and mC (grey) vary linearly with the temperature (from 20 - 90°C). According to our empirical observations in thermal baths, the change in fluorescence results slightly different when the protein is attached on the surface of MNPs. **Figure 8d-f** shows the fluorescence vs. temperature calibration curves for MNP-GFP and MNP-mC complexes. In the case of GFP (black), the fluorescence of System A shows a biphasic behaviour with a reduction of the 40% of its intensity from 20 to 40°C and another rapid decay from 60°C and decays rapidly over this temperature. System B and C show a progressive decrease of fluorescence observed in the 20-90°C range. In the case of MNP-mC (grey), it is system B the one that presents a biphasic behaviour with a decrease of 50% of fluorescence between 0-50°C and s second drop of 10% between 70-90°C, whereas System A and C displays a linear decay.

**Figure 8g-i** shows the local temperatures registered by GFP (black) or mC (grey) fluorescence loss after exposing MNPs-GFP or MNP-mC complexes to AMF with different frequency and field conditions for 5 min. The incubation conditions (MNPs-protein concentration, buffer, pH and time) were identical of those carried out when the calibration curves using global heating were obtained. In addition, no increase on the global temperature was observed while applying the AMF. Thus, the local temperature values were obtained by comparing the fluorescence loss with their respective calibration curves above mentioned **(Fig 8d-f)**. Both independent thermometers displayed compatible temperatures within a limit of 5 °C. The fluorescence reduction observed in system A indicates that the system generates local temperature between 35 and 65°C where the lowest temperature is obtained applying the field with the highest frequency and lower field. Although this system presents the lowest SAR in most of the conditions, temperatures up to 60°C are registered due to the close proximity of the thermometer to the surface of the MNPs. In the case of System B the higher is the frequency, the closer is the local temperature to 90°C.

In contrast to this trend, system C system presents an almost biphasic behaviour. At 100 kHz, the fluorescence decays to 80% what corresponds to a local temperature of 85°C. For frequencies equal or higher than 300 kHz, the registered fluorescence decreases indicating local temperatures close to 50-70°C.

Thus, the importance of determining the local temperature and not only the global temperature relies in the fact that, although there are 4- fold differences in SAR between systems A and B at the same AMF condition, the local temperatures reached only differ in 10 and 20°C. This is due to the difference in distance to the inorganic nucleus at which the GFP or mC is, much closer in the case of the System A than the System C.

In the light of these results, we have identified two ideal AMF conditions for the simultaneous and sequential activation of enzymatic reactions. Combining the three systems described above and applying an AMF 100 kHz/50 mT, it is possible to induce three different local temperatures at the surface of each system (**Table 3**). Alternatively, the combination of system B and C results ideal for selective activation using 100 kHz/50 mT and 388 kHz/10 mT (**Table 4**).

**Table 3:** Working example of a one-pot simultaneous activation by using MNPs that can be functionalized with different enzymes. They may operate at different temperatures between 25 and 85°C when attached to different MNPs.

| MNP | AMF | Local T |
|---|---|---|
| System A | | 50°C |

(continued)

| MNP | AMF | Local T |
|---|---|---|
| System B | 50 mT, 100 kHz | 25°C |
| System C | | 85°C |

**Table 4:** Working example of a one-pot sequential activation by using MNPs that can be functionalized with different enzymes. The local temperature of each system reaches quite different local temperatures at different AMF field conditions.

| MNP | AMF | Local T |
|---|---|---|
| System B | 50 mT, 100 kHz | 25 °C |
| System C | | 85 °C |
| System B | 10 mT, 388 kHz | 70 °C |
| System C | | 50 °C |

**EXAMPLE 7: Proof of concept of the generation of multiple local temperatures in one-pot using a mixture of two MNP-fluorescent proteins complexes**

As a proof of concept, we have tested a combination of two complexes (System B-GFP and System C-mC) mixed in the same reactor and exposed to two different AMFs: $AMF_1$= 100 kHz/50 mT; $AMF_2$= 388 kHz/10 mT. **Figure 9a** shows the local temperature registered for GFP and mC fluorescence reduction at AMF, conditions. It can be observed that the local temperature registered for each complex of the mixture is the same as the one observed in individual experiments of the previous example. It probes that a mixture of MNPs operating in the same reactor and AMF conditions is able to generate different local temperatures without interfering each other. **Figure 9b** compare these results with those obtained in each system at $AMF_2$. In this case, the local temperature of System B-GFP was risen up to 70 °C whereas the System C-mC resulted reduced to 50 °C, both similar to the ones observed in individual experiments. Besides, the temperature in the reactor remained constant at 17 $\pm$1 °C in both AMF conditions.

Therefore, the approach of the present invention related to generating at one or more different temperatures simultaneously for the simultaneous activation of enzymatic processes in a single-pot reactor by combining MNPs with different magnetic features and with different AMF, is supported by empirical evidence. Also, the approach of the present invention related to inducing maximum temperature at different AMF conditions for a sequential activation of thermally dependent enzymatic processes is supported by empirical evidence too.

**EXAMPLE 8: Testing catalytic effectiveness on Sucrose phosphorylase and alcohol dehydrogenase**

Next, the inventors assayed the functionalization of two different enzymes on the surface of the MNPs:
**a)** *Sucrose phosphorylase from Leuconostoc mesenteroids 2 (LmSP).* It is an enzyme that catalyzes the process to obtain $\alpha$ and glucose and 1 and phosphate from sucrose. This is an interesting intermediate as a "building block" for various products of industrial interest, both in cosmetics and in the food industry (e.g., cellobiose sweetener). This enzyme has an optimal temperature of 70°C. The challenge in this case is to achieve the efficient bioconversion of sucrose into $\alpha$- glucose and 1 and phosphate by minimizing the thermal decomposition of fructose, which is also a product of the phosphorylation reaction of sucrose. This enzyme will then be integrated into an enzymatic cascade to obtain the sweetener from cellobiose in a process that allows its profitable production in the food industry. The results are shown in **Table 5** below:

| Protein loading (µg /100 µg_MNP) | Protein bound (µg /100 µg_MNP) | Yield (%) | Specific activity (U/mg) | Catalytic effectiveness (%) | Number of trials |
|---|---|---|---|---|---|
| 50 | 20 ± 7 | 35 ± 6.3 | 113 ± 45.9 | 43 ± 3.3 | 2 |
| 100 | 44 ± 21 | 37 ± 9.8 | 119.5 ± 2.1 | 40.5* | 2 |
| 200 | 63 | 30.2 | 45 | 26.2 | 1 |
| 250 | 67 ± 25 | 26 ± 9.8 | 43.5 ± 30.4 | 19.9 ± 7.7 | 2 |

**Table 5.** Optimization of the immobilization of LSMP sucrose phosphorylase System A MNPs. % Yield: yield based on bound protein; Catalytic effectiveness (%): expressed activity of the enzyme bound to MNPs.

Similarly, as in the case of GFP shown above, the binding of this enzyme to MNPs is selective through the histidine tag, since when the immobilization is carried out in the presence of imidazole, no binding of the enzyme occurs. In turn, the binding is reversible since all the bound protein can be eluted after being incubated with imidazole.

Next, specificity and reversibility of the functionalization of LSMP in MNPs system A was assayed, as previously reported, using imidazole before or after the functionalization. The results are shown in **Table 6**.

**During immobilization:**

0.5 M imidazole in 10 mM MES buffer pH7

| Sample | Protein intended to load (µg /100 µg_MNP) | Protein actually loaded (µg /100 µg_MNP) | Protein bound (µg /100 µg_MNP) |
|---|---|---|---|
| *Lm*SP | 100 | 150 | ~0 |

**Post immobilization:**

1 M imidazole in 10 mM MES buffer pH7

| Sample | Protein bound (µg /100 µg_MNP) | Protein released (µg) | % Release |
|---|---|---|---|
| *Lm*SP-MNP | 27 | 28 | 100 |

The binding of *Lm*SP is specific and reversible

**Table 6.** Specificity and reversibility study.

**b) *Bacillus stearothermophilus* alcohol dehydrogenase (BsADH).** This ADH is a tetrameric enzyme of 150 kDa, thus containing four histidine tails on its surface with increased risk of aggregation of the MNPs during their functionalization. In addition, this enzyme catalyses a reaction of industrial interest as it is the interconversion between alcohols and aldehydes or ketones with the reduction of $NAD^+$ to NADH. The challenge in this case is that this enzyme's optimum temperature is 70°C and many reaction products (racemic alcohols used as "building blocks") and the cofactor (NAD) are heat sensitive. Thus, the final objective is to use this enzyme in combination with a mesophile format dehydrogenase (FDH) to be able to obtain enantiopure alcohols from ketoesters in one-pot by recycling the cofactor and without thermal decomposition of the substrate, the co-factor or the FDH.

**Table 7** shows that, in case of System A and using enzyme concentrations higher than 100 ug/ml and Co as divalent metal activity, the amount of enzyme bound to the MNPs is improved and MNPs aggregation is avoided during enzyme functionalization.

**Table 7.** Comparison of immobilization yields with respect to the amount of ADH protein and its activity. Characterization data of the hydrodynamic diameter are included after the functionalization of the MNPs. * Hydrodynamic diameter System A-Cu$^{+2}$=280 and System A-Co$^{+2}$ = 285. % Yield: yield based on bound protein; Catalytic effectiveness (%): expressed activity of the enzyme bound to MNPs.

| MNP | Protein Offered $_{(mg/mL)}$ | % Yield | % Catalytic effectiveness | Hydrodynamic Diameter (nm)* |
|---|---|---|---|---|
| System A-Cu$^{+2}$ | 100 | 100% | 1% | 575 |
| | 150 | 80% | 4% | 500 |
| | 200 | 85% | 9% | 430 |
| System A-Co$^{+2}$ | 100 | 70% | 40% | 330 |
| | 150 | 80% | 33% | 360 |
| | 200 | 80% | 30% | 345 |

## EXAMPLE 9: Defining the optimum AMF conditions

In order to offer a clearer landscape of the heating power of the different MNPs, **Figure 3** shows their SAR values dependence with frequency at a fixed H$_{MAX}$ of 16 mT and the dependence with the H$_{MAX}$ at low and high frequencies. Three possible scenarios (Figure 3A, B and C, respectively) can be identified:

1) At intermediate frequency (500 kHz) and low H$_{MAX}$ (16 mT), the MNPs show increasing values of SAR (System C < System A < System B) small for all the cases. This could be an interesting scenario to create simultaneous heating because small SAR implies small heating of the media although the temperature on the surfaces is much higher. Numerical values shown in **Table 8**.

**Table 8. Simultaneous heating.** SAR obtained with MNPs of different magnetic anisotropy for intermediate frequency conditions.

| AMF at medium frequency (491 kHz) | | | | |
|---|---|---|---|---|
| | H$_A$ (mT) | H$_{MAX}$ (mT) | H$_{MAX}$/H$_A$ | SAR (W/g) |
| System A | 27 | 16 | 0.59 | 59 |
| System B | 34 | 16 | 0.47 | 281 |
| System C | 41 | 16 | 0.39 | 91 |

2) At low frequencies (92 kHz) and high H$_{MAX}$ (60 mT), System C MNPs presents significantly higher SAR than the rest. Numerical values are shown in **Table 9**. This could be an interesting scenario to create sequential heating combining System B and C due to the large difference in their SAR values observed under this AMF setting.

**Table 9. Sequential heating.** SAR and surface temperature obtained with MNPs of different magnetic anisotropy field and different applied field for low frequency conditions.

| AMF high field intensity and low frequency (92 kHz) | | | | |
|---|---|---|---|---|
| | H$_A$ (mT) | H$_{MAX}$ (mT) | H$_{MAX}$/H$_A$ | SAR (W/g) |
| System B | 34 | 10 | 0.3 | 12 |
| | 34 | 20 | 0.6 | 38 |
| | 34 | 40 | 1.17 | 73 |
| | 34 | 60 | 1.8 | 95 |
| System C | 41 | 10 | 0.2 | 8 |
| | 41 | 20 | 0.5 | 40 |
| | 41 | 40 | 1 | 187 |
| | 41 | 60 | 1.5 | 303 |

3) At high frequencies (760 kHz) and low $H_{MAX}$ (16 mT), it is the System B sample the one with largest SAR. Numerical values in **Table 10.** This could be an interesting scenario to create sequential heating combining System B and C due to the large difference in their SAR values observed under this AMF setting.

**Table 10.** SAR obtained with MNPs of different magnetic anisotropy field and different applied field for high frequency conditions.

| AMF low field intensity and high frequency (760 kHz) | | | | |
|---|---|---|---|---|
| | $H_A$ (mT) | $H_{MAX}$ (mT) | $H_{MAX}/H_A$ | SAR (W/g) |
| System B | 34 | 5 | 0.15 | 46 |
| | 34 | 10 | 0.29 | 97 |
| | 34 | 16 | 0.47 | 529 |
| | 34 | 20 | 0.59 | 568 |
| System C | 41 | 5 | 0.12 | 0 |
| | 41 | 10 | 0.24 | 41 |
| | 41 | 16 | 0.39 | 269 |
| | 41 | 20 | 0.50 | 297 |

Thus, the best condition to perform a sequential activation using the different heating power of Systems B and C at different AMFs can be combining scenarios 2 and 3.

Other examples of simultaneous activation are represented below:

**Table 11. Simultaneous SAR** obtained with MNPs of different magnetic anisotropy field and different applied field for low frequency conditions.

| AMF varying field intensity at low frequency (92 kHz) | | | | |
|---|---|---|---|---|
| | $H_A$ (mT) | $H_{MAX}$ (mT) | $H_{MAX}/H_A$ | SAR (W/g) |
| System A | 27 | 10 | 0.4 | 3 |
| | 27 | 20 | 0.7 | 22 |
| | 27 | 40 | 1.5 | 44 |
| | 27 | 60 | 2.2 | 56 |
| System B | 34 | 10 | 0.3 | 12 |
| | 34 | 20 | 0.6 | 38 |
| | 34 | 40 | 1.17 | 73 |
| | 34 | 60 | 1.8 | 95 |
| System C | 41 | 10 | 0.2 | 8 |
| | 41 | 20 | 0.5 | 40 |
| | 41 | 40 | 1 | 187 |
| | 41 | 60 | 1.5 | 303 |

**Table 12.** Simultaneous SAR obtained with MNPs of different magnetic anisotropy field and different applied field for high frequency conditions.

| AMF varying field intensity at high frequency ( 760 kHz) | | | | |
|---|---|---|---|---|
| | $H_A$ (mT) | $H_{MAX}$ (mT) | $H_{MAX}/H_A$ | SAR (W/g) |
| System A | 27 | 5 | 0.19 | 58 |
| | 27 | 10 | 0.37 | 59 |
| | 27 | 16 | 0.59 | 98 |
| | 27 | 20 | 0.74 | 126 |
| System B | 34 | 5 | 0.15 | 46 |
| | 34 | 10 | 0.29 | 97 |
| | 34 | 16 | 0.47 | 529 |
| | 34 | 20 | 0.59 | 568 |
| System C | 41 | 5 | 0.12 | 0 |
| | 41 | 10 | 0.24 | 41 |
| | 41 | 16 | 0.39 | 269 |
| | 41 | 20 | 0.50 | 297 |

In order to extract an empirical value for the anisotropy field of the different systems of MNPs, we have fitted the experimental values of SAR obtained from calorimetric measurements with a sigmoidal Hill function. Considering the MNPs as weakly interacting systems, the center of the sigmoid can be approximated to the anisotropy field of the sample. The results of the fitting are included in **Table 13** and **Figure 11.** As shown, the anisotropy field values obtained grow with the volume of the MNPs.

**Table 13.** $H_A$ values obtained from the fitting of SAR growth with $H_{MAX}$.

| MNP | Hill sigmoid center (mT) |
|---|---|
| System A | 27 |
| System B | 34 |
| System C | 41 |

### EXAMPLE 10: Reaction media temperature

**Figure 12** shows the increase of temperature registered in the media when diluted colloids of MNPs in the systems A, B and C are exposed to their maximum SAR AMF conditions. The Fe concentration were progressively reduced from 1 to 0.005 mg mL$^{-1}$. At the operative concentrations (<0.1 mg$_{Fe}$ mL$^{-1}$) the temperature of the dispersion media was increased bellow 1 Celsius degree for AMF applications periods of 15 minutes. These results prove that, using diluted dispersions of MNPs, it is possible to increase the temperature at the surface of MNPs minimizing the global heating of the reaction media what made feasible the combination of different MNPs system for the thermal control of different enzymes@MNPs derivatives mixed in the same reactor.

### EXAMPLE 11: Sequential activation in a one-pot cascade

**Figure 13** shows an example of the on-demand regulation of mesophilic enzymes working together in a one-pot cascade system. In this case, the challenge is to selectively turn ON/OFF enzymes with similar OT during the cascade operation. We selected a one-pot multi-enzyme cascade to synthesize stereopure isoquinolones, which are precursors for many active pharmaceutical ingredients (APIs). This cascade requires the orchestration of thiaminediphosphate dependent carboligase (TC), ω-transaminase (TA) and norcroclaurine synthase (NCS). When TC and TA are simultaneously active, product yields dramatically decay due their unpaired substrate specificities that limits the products yield, derailing the synthetic route to undesired by-products.

To selectively regulate this cell-free biosynthetic pathway on-demand, a one-pot reactor will be operated with low reaction media temperatures (10°C) under AMF sequences with different settings that selectively trigger target enzymes by local heating (25-37°C). Firstly, each enzyme should be attached to a different MNPs system with specific optimum AMF conditions (TC-MNP1, TA-MNP2, NCS-MNP2). Then, TC-MNP1 will be selectively activated under its own optimum AMF (AMF1), while TA-MNP2 and NCS-MNP2 remain inactive since AMF1 does not results suitable for MNP2 and MNP2 at their enzyme-MNP interfaces. When the first reaction is completed, AMF settings are changed (AMF2), so TA-MNP2 and NCS-MNP2 become active while local gradient surrounding TC remains constant. This AMF sequence avoids TA and TC to compete towards the same substrate, increasing the product yields. Once maximum product yield is reached; turning OFF the AMF would inactivate the three enzymes so they could be switched ON again in a subsequent reaction cycle. This would tackle the system reuse that otherwise cannot be accomplished. A toolbox of MNPs able to reach similar temperature gradients but triggered by different AMF settings will be crucial to face this challenge.

**EXAMPLE 12: Simultaneous activation of a one-pot cascades**

In the case of simultaneous enzyme nanoactuation by magnetic heating, several cases studies were selected that involve different challenges that could not be tackled through global heating of the reaction media.

a)- **Case study 1.** *Combining a thermophilic and a mesophilic enzyme ensuring stability of thermolabile co-factors.* **Figure 14a** represents the asymmetric reduction of ketoesters catalysed by NADH-dependent thermophile dehydrogenase (ADHT) ($T_{OPT}$ = 70 °C) to obtain enantiopure precursors for many APIs. This bioprocess shows a dramatic trade-off between the high temperature needed to function the ADHT at maximum speed, and the low thermal stability of redox cofactors (NADH) mandatory for the enzyme activity. Besides, the differences between $T_{OPT}$ between ADHT and a mesophile NADH recycling partner (e.g formate dehydrogenase, FDH) limit the cofactor turnover frequency, negatively impacting on system productivity. A maximum productivity should be obtained if ADHT is able to work at its optimal temperature by local magnetic heating through its binding to any of the MNP systems developed while the global temperature of the reaction vessel is low enough to avoid denaturation of FDH or decomposition of the thermolabile cofactor.

b) **Case study 2.** *Combining two thermophilic with a mesophilic enzyme ensuring stability of thermolabile co-factors.* **Figure 14b** shows a coupled multi-enzyme process for the obtention of hydroxy-propionaldehyde enantiopure derivatives which are important components to prepare "building blocks" (lactones, amino alcohols, etc.) in the synthesis of a large number of cosmetics, food preservants, lubricants and drugs. The multi-enzyme cascade includes an alcohol dehydrogenase (ADH) from *B. stearothermophilus* that is responsible for the selective oxidation of 1,3-propanediol derivatives. Since this enzyme is dependent on NADH as a cofactor, a NADH-oxidase (NOX) from *T. thermophilus* is also included in the reaction scheme for a continuous in situ regeneration of the cofactor. NOX generates hydrogen peroxide as a by-product to regenerate the NADH consumed by ADH. Since $H_2O_2$ is a powerful oxidant and can denature ADH, a mesophilic catalase (CAT) is also added to the reaction scheme to convert the generated $H_2O_2$ into water. ADH and NOX are both thermophilic enzymes but with different optimal temperatures (65°C and 90°C, respectively). Thus, in this case a maximum productivity should be obtained if both thermophilic enzymes are able to work simultaneously at their optimal temperature under the same AMF setting through their binding to the most adequate dual combination of the MNP systems developed. In addition, during their AMF-induced differential thermal activation the global temperature of the reaction vessel has to be low enough to avoid denaturation of CAT ($T_{OPT}$ = 30°C) or decomposition of the thermolabile cofactor.

c)- **Case study 3.** *High-temperature operation of a three-enzyme cascade with reduced thermal decomposition of by-product.* **Figure 14c** represents a three-enzyme cascade for high temperature sucrose conversion into industrial important cellobiose derivatives without fructose decomposition. It involves sucrose phosphorylase (SP), glucose isomerase (GI) and cellobiose phosphorylase (CBP) with different optimal temperatures and thus thermal stabilities. This fact hampers working with the three enzymes at their maximum efficiency in the same vessel using conventional global heating systems. Furthermore, sub-optimal cascade operation drives the accumulation of fructose as by-product that under high temperatures is decomposed, browning the product solution, which is unsuitable for further use (e.g., in cosmetics or food) unless excessive additional purification steps are applied. Thus, in this case a maximum productivity is expected under an AMF operation setting where MNPs hybrids from SP from *L. mesenteroides* ($T_{OPT}$ = 70°C), GI from *S. murinus* ($T_{OPT}$ = 85°C) and CBP from *C. thermocellum* ($T_{OPT}$ = 60 °C) could work at their respective optimal temperature while the global temperature of the reaction media is low enough to avoid thermal decomposition of fructose if it is produced even in an expected low yield.

**EXAMPLE 13: Metal affinity conjugation of TTADH and BsADH proteins to MNPs**

**Materials and Methods:**

*1. Water transfer of hydrophobic magnetic nanoparticles.*

The transference was performed as in previous occasions, adding 225 mg of PMAO to 98 mL of chloroform, then, the equivalent to 10 mg of iron in nanoparticles suspended in 2 mL of chloroform were added drop by drop and the mixture was stirred under ultrasonic bath for 15 min. The solvent was rotoevaporated until 5-10 mL. Nanoparticles were resuspended in 20 mL of NaOH 0.05 M and the sample was rotoevaporated again until complete disappearance of foam. NPs were then filtered on syringe filters of 0.22 $\mu$m. The excess of unbound polymer was removed by centrifugations at 70000 x g at 14°C for 8 h. NPs were then redispersed in water and a gel electrophoresis was performed to check the complete elimination of the polymer excess.

*2. Preparation of stock solution of NTA-Me$^{2+}$ complex.*

Briefly, we mixed 25 mM of NTA and 30 mM of $CoCl_2$ or CuSO4 in 80 mL of borate buffer saline (BBS) solution (50 mM, pH 8.0). Then the pH was adjusted to 10.5 to precipitate the excess of $Me^{2+}$ ions, the complex was centrifugated (15 min 5000 RPM) and the final pH was adjusted to 9.0.

*3. One pot functionalization with LysNTA-Me$^{2+}$ complex and PEG5000.*

32 $\mu$mols of stock NTA-Me$^{2+}$/mg$_{Fe}$ were mixed with 18.5 $\mu$mols of PEG5000/mg$_{Fe}$. The functionalization was performed with 0.5 mg of Fe in each microtube adjusting to a final volume of 1500 $\mu$l. To activate the carboxyl groups, EDC (6.36 mg/mg Fe) was dissolved in 20 $\mu$l of SSB buffer at pH 9 and was added in the same reaction 10 $\mu$L at time 0 and 10 $\mu$L after 30 minutes, letting the mixture react at 37 °C and stirred at 800 RPMs for three hours and 30 minutes in total. Then, eight washes using ultra-centrifugal filters (Amicon, Millipore, 100 kDa) were performed to eliminate unreacted reagents.

*4 - Metal affinity conjugation of TtADH and BsADH proteins to MNPs.*

The Alcohol dehydrogenase from *Thermus thermophilus* (TtADH) and Alcohol dehydrogenase from *Bacillus stearothermophilus (BsADH)* conjugation was carried on by incubating 100 $\mu$g$_{protein}$ with 100 $\mu$g$_{Fe}$ in phosphate buffered saline (dPBS, w/o Ca or Mg) adjusting the final volume to 250 $\mu$L. The reaction was maintained under continuous stirring at 37°C for 1 hours protected from light. After this time, the microtubes were centrifuged twice during 1 hour at 25000 x g and 4 °C, on the end of each centrifugation 200 $\mu$L of supernatants were collected and 200 $\mu$L of new buffer dPBS were added, sonicating the MNPs during 10 sec. The supernatants were stored at 4°C for further analysis.

*5 - Protein concentration determination*

The protein concentration was determined by Bradford assay using Bovine Serum Albumin (BSA, Sigma Aldrich, USA) as standard. Briefly, 150 $\mu$L of each standard or unknown sample were pipetted into the appropriate microplate wells. Then, 150 $\mu$L of the Coomassie Plus Reagent (Thermofisher Scientific, USA) were added to each well and mixed for 30 seconds. The plate was incubated for 10 minutes at room temperature, then the absorbance at 595nm was measured using a Thermofisher scientific plate reader.

*6 - Protein yield*

The amount of protein bound to the carrier is expressed as the difference between the amount of protein offered in reaction and the amount of protein unbound in the solution (Equation 2).

$$P_{immob} = P_{offered} - P_{sup} \qquad (2)$$

Where: $P_{inmob}$ represents the amount of protein bound to the MNPs, $P_{offered}$ is the amount of protein offered in reaction and $P_{sup}$ is amount of unbound protein, found in the in the supernatant and the washing steps.

The immobilization yield (Y) was calculated as the ratio between the amount of protein immobilized on to the carrier and the amount of protein offered to the carrier (Equation 3).

$$Y(\%) = \frac{P_{immob}}{P_{offered}} \times 100 \qquad (3)$$

*7 - Local heating determination by a enzymatic activity.*

∘ *Global heating effect on TtADH*

The samples were diluted to contain 3,5μg/mL of TtADH and the dependence of its enzymatic activity with temperature was studied by heating the solution globally using a thermomixer. Each protein aliquot was incubated for 5 minutes at different temperature in a thermomixer under shaking at 350 rpm. Then, the activity assay was recorded as follow: 200 μL of reaction mixture containing 0.2 mM NADH, 5% of isopropanol and 10 mM of ethyl acetoacetate in 100 mM sodium phosphate at pH 7 were added to 5 μL of enzymatic solution at room temperature, and the decrease in the absorbance was monitored at 340 nm ($\varepsilon$NADH = 6.22 mM$^{-1}$ x cm$^{-1}$). One unit of activity corresponds to the amount of enzyme needed to consume 1 μmol of NADH in one minute.

∘ *Effect of the application of an alternating magnetic field to the conjugated TtADH activity.*

Samples were diluted to contain 3.5 μg/mL of TtADH and placed into an AMF inductor. The AMF at different settings (frequency and field intensity combinations) was applied for 5 minutes. After the treatment, the activity assay was monitored and the local temperature registered was inferred using an Arrhenius plot (Equation 4)

$$k = Ae^{\frac{-E_a}{RT}}$$
(Equation 4)

where

- k is the rate constant (frequency of collisions resulting in a reaction),
- A is the pre-exponential factor.
- Ea is the activation energy for the reaction (in the same units as RT),
- R is the universal gas constant,
- T is the absolute temperature (in degrees Kelvin ).

**Figure 15** shows that the optimal temperature for TtADH is 70°C (panel 15A). From these data, an Arrhenius plot was derived and used as calibration curve (panel 15B) for determining the local temperature of the TtADH conjugates under AMF conditions. From panel 15C it is possible to observe that an overactivation was observed when incubating the conjugates under AMF, indeed, it was possible to observe an activity comparable to the one obtained in a range of 44-50°C.

**REFERENCES**

[1] Munoz-Menendez et al. Towards improved magnetic fluid hyperthermia: major-loops to diminish variations in local heating Phys. Chem. Chem. Phys., 2017,19, 14527-14532.
[2] Costo, R., Heinke, D., Grüttner, C. et al. Improving the reliability of the iron concentration quantification for iron oxide nanoparticle suspensions: a two-institutions study. Anal Bioanal Chem, 2019, 411, 1895-1903.

**Claims**

**1.** A process to carry out two or more enzymatic reactions in a reaction medium, comprising:

   a) providing a system which in turn comprises at least two substantially homogeneous and colloidal populations of magnetic nanoparticles (MNPs), **characterized in that** each of the populations of MNPs are functionalized with at least one different enzyme,
   **characterized in that** each of the populations of MNPs have a different magnetic anisotropy capable of dissipating local thermal energy on the surface of each of the populations of MNPs upon application of an external alternating magnetic field (AMF) sufficient to activate each of the enzymes functionalized on said MNPs, and
   b) applying one or more external AMFs to the system of a) to produce a simultaneous or sequential activation of the enzymes functionalized on the surface of each of the populations of MNPs so that the enzymatic reactions are carried out,

wherein during step b) the reaction media is kept at a significantly lower temperature than the lowest optimum temperature of the enzymes functionalized on the surface of each of the populations of MNPs.

2.  The process according to claim 1, wherein the MNPs of each of the populations of MNPs of step a) are further **characterized by** having an average particle diameter of less than 200 nm, preferably between 5 and 50 nm.

3.  The process of any of claims 1 or 2, wherein the MNPs of each of the populations of MNPs of step a) are further **characterized by** having a core selected from the group consisting of iron oxide, magnetite or maghemite.

4.  The process of any of claims 1 to 3 wherein the magnetic anisotropy capable of dissipating local thermal energy on the surface of each of the populations of MNPs is within the ranges of 34 mT $\pm$ 50%, preferably 34 mT $\pm$ 25%.

5.  The process of any of claims 1 to 4, wherein the MNPs of each of the populations of MNPs of step a) are further **characterized by** being coated with at least hydroxyl, carboxyl, carbonyl, amino, thiol, azide, or alkyne functional groups.

6.  The process of any of claims 1 to 5, wherein the coating is performed with polyacrylic acid (PAA), dimercaptosuccinic acid (DMSA) and/or poly(maleic anhydride-alt-1-octadecene (PMAO), or with any combination thereof.

7.  The process of any of claims 1 to 6, wherein the MNPs of each of the populations of MNPs of step a) are further **characterized by** being functionalized with at least a chelating agent and a divalent metal ion.

8.  The process of any of claims 1 to 7, wherein the chelating agent comprises nitriloacetic acid derivatives (NTA) and the divalent metal ions is selected from the group consisting of $Cu^{2+}$, $Co^{2+}$, $Ni^{2+}$.

9.  The process of any of claims 1 to 8, wherein the enzymes functionalized in the surface of the MNPs of each of the populations of MNPs of step a) are selected from the group consisting of Sucrose phosphorylase from *Leuconostoc mesenteroids,* Bacillus stearothermophilus alcohol dehydrogenase, *Thermus thermophilus* alcohol dehydrogenase, amylase from *Bacillus licheniformis,* glucose isomerase from *Suncus murinus,* cellobiose phosphorylase from *Clostridium thermocellum,* L-aspartate oxidase from *Sulfolobus tokodaii, Bacillus megaterium* or *Chromobacterium violaceum* transaminases, *Acetobacter pasteurianus* pyruvate decarboxylase, collagenase from *Bacillus sp,* hyaluronidase from *Thermasporomyces composti, Thermus thermophilus* lipase and NADH-oxidase, horseradish peroxidase, *Thermus thermophilus, Saccharolobus shibatae* uracil phosphoryl transferase, or *Escherichia coli* cytosine deaminase.

10. The process of any of claims 1 to 9, wherein the magnetic anisotropy of each of the populations of MNPs is within the range of 34 mT $\pm$ 50%, and the external AMF is **characterized by** a frequency range between 50 and 800 kHz and a magnetic flux density of between 5 and 70 mT, wherein, preferably, the ratio between the magnetic flux density and the magnetic anisotropy is greater than 0.4, preferably greater than 0.5.

11. The process of any of claims 1 to 10, wherein the at least two substantially homogeneous and colloidal populations of magnetic nanoparticles (MNPs) comprised in the system of a) have an iron oxide core and are selected from the group consisting of:

    i) MNPs **characterized in that** each of the MNPs further have an average particle diameter of between 8-15 nm, are coated with DMSA, functionalized with NTA and $Cu^{2+}$, and have a magnetic anisotropy of 27 mT;
    ii) MNPs **characterized in that** each of the MNPs further have an average particle diameter of between 18-25 nm, are coated with PMAO, functionalized with NTA and $Cu^{2+}$, and have a magnetic anisotropy of 34 mT; or
    iii) MNPs **characterized in that** each of the MNPs further have an average particle diameter of between 25-35 nm, are coated with PAA, functionalized with NTA and $Cu^{2+}$, and have a magnetic anisotropy of 41 mT;

    and wherein the one or more AMF applied in b) are selected from the group consisting of:

    i) magnetic flux density range of 40-50 mT and frequency range of 80-120 kHz,
    ii) magnetic flux density range of 5-20 mT, and frequency range of 300-400 kHz,
    iii) magnetic flux density range of 10-20 mT, and frequency range of 400-500 kHz,
    iv) magnetic flux density range of 50-60 mT, and frequency range of 50-100 kHz,
    v) magnetic flux density range of 50-60 mT, and frequency range of 50-100 kHz, or

vi) magnetic flux density range of 20-30 mT, and frequency range of 600-800 kHz.

vii) magnetic flux density range of 5-20 mT, and frequency range of 600-800 kHz.

12. A **system** comprising at least two substantially homogeneous and colloidal populations of magnetic nanoparticles (MNPs), **characterized in that** each of the populations of MNPs are functionalized with at least one different enzyme, and **characterized in that** each of the populations of MNPs have a different magnetic anisotropy capable of dissipating local thermal energy on the surface of each of the population of MNPs upon application of an external alternating magnetic field (AMF) sufficient to activate each of the enzymes functionalized on said MNPs.

13. The system of claim 12, wherein the at least two substantially homogeneous and colloidal populations of magnetic nanoparticles (MNPs) comprised in the system of a) have an iron oxide core and are selected from the group consisting of:

i) MNPs **characterized in that** each of the MNPs further have an average particle diameter of between 8-15 nm, are coated with DMSA, functionalized with NTA and $Cu^{2+}$, and have a magnetic anisotropy of 27 mT;

ii) MNPs **characterized in that** each of the MNPs further have an average particle diameter of between 18-25 nm, are coated with PMAO, functionalized with NTA and $Cu^{2+}$, and have a magnetic anisotropy of 34 mT; or

iii) MNPs **characterized in that** each of the MNPs further have an average particle diameter of between 25-35 nm, are coated with PAA, functionalized with NTA and $Cu^{2+}$, and have a magnetic anisotropy of 41 mT.

14. *In vitro* use of the system according to any of claims 12 or 13 to carry out the enzymatic process of any of claims 1 to 11.

## Patentansprüche

1. Prozess zum Vornehmen zweier oder mehrerer enzymatischen Reaktionen in einem Reaktionsmedium, umfassend:

a) das Bereitstellen eines Systems, welches wiederum mindestens zwei im Wesentlichen homogene und kolloidale Populationen von magnetischen Nanoteilchen (MNP) umfasst, **dadurch gekennzeichnet, dass** jede der Populationen von MNP mit mindestens einem unterschiedlichen Enzym funktionalisiert ist, **dadurch gekennzeichnet, dass** jede der Populationen von MNP eine unterschiedliche magnetische Anisotropie aufweist, welche in der Lage ist, lokale thermische Energie auf der Oberfläche jeder der Populationen von MNP nach dem Anlegen eines äußeren magnetischen Wechselfelds (AMF) zu zerstreuen, welches ausreichend ist, um jedes der auf den genannten MNP funktionalisierten Enzyme zu aktivieren, und

b) das Anlegen von einem oder mehreren äußeren AMF auf das System aus a), um eine gleichzeitige oder sequenzielle Aktivierung der auf der Oberfläche jeder der Populationen von MNP funktionalisierten Enzyme zu erzeugen, sodass die enzymatischen Reaktionen vorgenommen werden,

wobei während Schritt b) das Reaktionsmedium bei einer bedeutend niedrigeren Temperatur als die niedrigste optimale Temperatur der auf der Oberfläche jeder der Populationen von MNP funktionalisierten Enzyme gehalten wird.

2. Prozess nach Anspruch 1, wobei die MNP jeder der Populationen von MNP aus Schritt a) zusätzlich **dadurch gekennzeichnet sind, dass** sie einen mittleren Teilchendurchmesser von weniger als 200 nm, vorzugsweise zwischen 5 und 50 nm, aufweisen.

3. Prozess nach einem der Ansprüche 1 oder 2, wobei die MNP jeder der Populationen von MNP aus Schritt a) zusätzlich **dadurch gekennzeichnet sind, dass** sie einen Kern ausgewählt aus der Gruppe bestehend aus Eisenoxid, Magnetit oder Maghemit aufweisen.

4. Prozess nach einem der Ansprüche 1 bis 3, wobei die magnetische Anisotropie, welche in der Lage ist, lokale thermische Energie auf der Oberfläche jeder der Populationen von MNP zu zerstreuen, innerhalb der Bereiche von 34 mT $\pm$ 50 %, vorzugsweise 34 mT $\pm$ 25 %, liegt.

5. Prozess nach einem der Ansprüche 1 bis 4, wobei die MNP jeder der Populationen von MNP aus Schritt a) zusätzlich **dadurch gekennzeichnet sind, dass** sie mindestens mit funktionellen Hydroxyl-, Carboxyl-, Carbonyl-, Amino-, Thiol-, Azid- oder Alkingruppen beschichtet sind.

6. Prozess nach einem der Ansprüche 1 bis 5, wobei die Beschichtung mit Polyacrylsäure (PAA), Dimercaptobernsteinsäure (DMSA) und/oder Poly(maleinsäureanhydrid-alt-1-octadecen) (PMAO), oder mit einer Kombination derselben, durchgeführt wird.

7. Prozess nach einem der Ansprüche 1 bis 6, wobei die MNP jeder der Populationen von MNP aus Schritt a) zusätzlich **dadurch gekennzeichnet sind, dass** sie mit mindestens einem Chelator und einem zweiwertigen Metallion funktionalisiert sind.

8. Prozess nach einem der Ansprüche 1 bis 7, wobei der Chelator Nitriloessigsäure-Derivative (NTA) umfasst und das zweiwertige Metallion aus der Gruppe bestehend aus $Cu^{2+}$, $Co^{2+}$, $Ni^{2+}$ ausgewählt wird.

9. Prozess nach einem der Ansprüche 1 bis 8, wobei die auf der Oberfläche der MNP jeder der Populationen von MNP aus Schritt a) funktionalisierten Enzyme aus der Gruppe bestehend aus Saccharosephosphorylase aus *Leuconostoc mesenteroids,* Alkoholdehydrogenase aus *Bacillus stearothermophilus,* Alkoholdehydrogenase aus *Thermus thermophilus,* Amylase aus *Bacillus licheniformis,* Glucoseisomerase aus *Suncus murinus,* Cellobiosephosphorylase aus *Clostridium thermocellum,* L-Aspartatoxidase aus *Sulfolobus tokodaii,* Transaminasen aus *Bacillus megaterium* oder *Chromobacterium violaceum,* Pyruvatdecarboxylase aus *Acetobacter pasteurianus,* Collagenase aus *Bacillus sp,* Hyaluronidase aus *Thermasporomyces composti,* Lipase und NADH-Oxidase aus *Thermus thermophilus,* Meerrettichperoxidase, Uracilphosphoryltransferase aus *Thermus thermophiles* und *Saccharolobus shibatae,* oder Cytosindeaminase aus *Escherichia coli* ausgewählt werden.

10. Prozess nach einem der Ansprüche 1 bis 9, wobei die magnetische Anisotropie jeder der Populationen von MNP innerhalb des Bereiches von 34 mT $\pm$ 50 % liegt und das äußere AMF durch einen Frequenzbereich zwischen 50 und 800 kHz und eine magnetische Flussdichte zwischen 5 und 70 mT gekennzeichnet ist, wobei, vorzugsweise, das Verhältnis zwischen der magnetischen Flussdichte und der magnetischen Anisotropie größer als 0,4, vorzugsweise größer als 0,5, ist.

11. Prozess nach einem der Ansprüche 1 bis 10, wobei die mindestens zwei im Wesentlichen homogenen und kolloidalen Populationen von magnetischen Nanoteilchen (MNP), welche im System aus a) umfasst sind, einen Eisenoxidkern aufweisen und aus der Gruppe ausgewählt werden, welche aus Folgenden besteht:

   i) MNP, **dadurch gekennzeichnet, dass** jedes der MNP zusätzlich einen mittleren Teilchendurchmesser zwischen 8-15 nm aufweist, mit DMSA beschichtet ist, mit NTA und $Cu^{2+}$ funktionalisiert ist und eine magnetische Anisotropie von 27 mT aufweist;
   ii) MNP, **dadurch gekennzeichnet, dass** jedes der MNP zusätzlich einen mittleren Teilchendurchmesser zwischen 18-25 nm aufweist, mit PMAO beschichtet ist, mit NTA und $Cu^{2+}$ funktionalisiert ist und eine magnetische Anisotropie von 34 mT aufweist; oder
   iii) MNP, **dadurch gekennzeichnet, dass** jedes der MNP zusätzlich einen mittleren Teilchendurchmesser zwischen 25-35 nm aufweist, mit PAA beschichtet ist, mit NTA und $Cu^{2+}$ funktionalisiert ist und eine magnetische Anisotropie von 41 mT aufweist;

   und wobei das eine oder die mehreren in b) angelegten AMF aus der Gruppe ausgewählt werden, welche aus Folgenden besteht:

   i) einem magnetischen Flussdichtebereich von 40-50 mT und einem Frequenzbereich von 80-120 kHz,
   ii) einem magnetischen Flussdichtebereich von 5-20 mT und einem Frequenzbereich von 300-400 kHz,
   iii) einem magnetischen Flussdichtebereich von 10-20 mT und einem Frequenzbereich von 400-500 kHz,
   iv) einem magnetischen Flussdichtebereich von 50-60 mT und einem Frequenzbereich von 50-100 kHz,
   v) einem magnetischen Flussdichtebereich von 50-60 mT und einem Frequenzbereich von 50-100 kHz, oder
   vi) einem magnetischen Flussdichtebereich von 20-30 mT und einem Frequenzbereich von 600-800 kHz.
   vii) einem magnetischen Flussdichtebereich von 5-20 mT und einem Frequenzbereich von 600-800 kHz.

12. System umfassend mindestens zwei im Wesentlichen homogene und kolloidale Populationen von magnetischen Nanoteilchen (MNP), **dadurch gekennzeichnet, dass** jede der Populationen von MNP mit mindestens einem unterschiedlichen Enzym funktionalisiert ist, und **dadurch gekennzeichnet, dass** jede der Populationen von MNP eine unterschiedliche magnetische Anisotropie aufweist, welche in der Lage ist, lokale thermische Energie auf der Oberfläche jeder der Populationen von MNP nach dem Anlegen eines äußeren magnetischen Wechselfelds (AMF) zu zerstreuen, welches ausreichend ist, um jedes der auf den genannten MNP funktionalisierten Enzyme zu aktivieren.

**13.** System nach Anspruch 12, wobei die mindestens zwei im Wesentlichen homogenen und kolloidalen Populationen von magnetischen Nanoteilchen (MNP), welche im System aus a) umfasst sind, einen Eisenoxidkern aufweisen und aus der Gruppe ausgewählt werden, welche aus Folgendem besteht:

i) MNP, **dadurch gekennzeichnet, dass** jedes der MNP zusätzlich einen mittleren Teilchendurchmesser zwischen 8-15 nm aufweist, mit DMSA beschichtet ist, mit NTA und $Cu^{2+}$ funktionalisiert ist und eine magnetische Anisotropie von 27 mT aufweist;

ii) MNP, **dadurch gekennzeichnet, dass** jedes der MNP zusätzlich einen mittleren Teilchendurchmesser zwischen 18-25 nm aufweist, mit PMAO beschichtet ist, mit NTA und $Cu^{2+}$ funktionalisiert ist und eine magnetische Anisotropie von 34 mT aufweist; oder

iii) MNP, **dadurch gekennzeichnet, dass** jedes der MNP zusätzlich einen mittleren Teilchendurchmesser zwischen 25-35 nm aufweist, mit PAA beschichtet ist, mit NTA und $Cu^{2+}$ funktionalisiert ist und eine magnetische Anisotropie von 41 mT aufweist.

**14.** In-vitro-Verwendung des Systems nach einem der Ansprüche 12 oder 13, zum Vornehmen des enzymatischen Prozesses nach einem der Ansprüche 1 bis 11.

**Revendications**

**1.** Procédé pour effectuer deux ou plusieurs réactions enzymatiques dans un milieu réactionnel, comprenant :

a) fournir un système qui comprend à son tour au moins deux populations sensiblement homogènes et colloïdales de nanoparticules magnétiques (MNP), **caractérisé en ce que** chacune des populations de MNP est fonctionnalisée avec au moins une enzyme différente,
**caractérisé en ce que** chacune des populations de MNP présente une anisotropie magnétique différente capable de dissiper l'énergie thermique locale sur la surface de chacune des populations de MNP lors de l'application d'un champ magnétique alternatif (AMF) externe suffisant pour activer chacune des enzymes fonctionnalisées sur lesdites MNP, et
b) appliquer un ou plusieurs AMF externes au système de a) pour produire une activation simultanée ou séquentielle des enzymes fonctionnalisées sur la surface de chacune des populations de MNP de sorte que les réactions enzymatiques sont effectuées,

dans lequel, pendant l'étape b), le milieu réactionnel est maintenu à une température significativement inférieure à la température optimale la plus basse des enzymes fonctionnalisées sur la surface de chacune des populations de MNP.

**2.** Procédé selon la revendication 1, dans lequel les MNP de chacune des populations de MNP de l'étape a) sont **caractérisées en outre par** le fait d'avoir un diamètre de particule moyen de moins de 200 nm, de préférence compris entre 5 et 50 nm.

**3.** Procédé selon l'une quelconque des revendications 1 ou 2, dans lequel les MNP de chacune des populations de MNP de l'étape a) sont **caractérisées en outre par** le fait d'avoir un noyau choisi dans le groupe constitué de l'oxyde de fer, la magnétite ou la maghémite.

**4.** Procédé selon l'une quelconque des revendications 1 à 3, dans lequel l'anisotropie magnétique capable de dissiper l'énergie thermique locale sur la surface de chacune des populations de MNP se trouve dans les intervalles de 34 mT $\pm$ 50 %, de préférence 34 mT $\pm$ 25 %.

**5.** Procédé selon l'une quelconque des revendications 1 à 4, dans lequel les MNP de chacune des populations de MNP de l'étape a) sont **caractérisées en outre par** le fait d'être recouvertes au moins de groupes fonctionnels hydroxyle, carboxyle, carbonyle, amino, thiol, azide, ou alcyne.

**6.** Procédé selon l'une quelconque des revendications 1 à 5, dans lequel le recouvrement est réalisé avec du poly(acide acrylique) (PAA), de l'acide dimercaptosuccinique (DMSA) et/ou du poly(anhydride maléique-alt-1-octadécène) (PMAO), ou avec toute combinaison de ceux-ci.

**7.** Procédé selon l'une quelconque des revendications 1 à 6, dans lequel les MNP de chacune des populations de MNP de l'étape a) sont **caractérisées en outre par** le fait d'être fonctionnalisées avec au moins un agent chélateur et un ion métallique divalent.

**8.** Procédé selon l'une quelconque des revendications 1 à 7, dans lequel l'agent chélateur comprend des dérivés de l'acide nitriloacétique (NTA) et les ions métalliques divalents sont choisis dans le groupe constitué de $Cu^{2+}$, $Co^{2+}$, $Ni^{2+}$.

**9.** Procédé selon l'une quelconque des revendications 1 à 8, dans lequel les enzymes fonctionnalisées dans la surface des MNP de chacune des populations de MNP de l'étape a) sont choisies dans le groupe constitué de saccharose phosphorylase de *Leuconostoc mesenteroids,* alcool déshydrogénase de *Bacillus stearothermophilus,* alcool déshydrogénase de *Thermus thermophilus,* amylase de *Bacillus licheniformis,* glucose isomérase de *Suncus murinus,* cellobiose phosphorylase de *Clostridium thermocellum,* L-aspartate oxydase de *Sulfolobus tokodaii,* transaminases de *Bacillus megaterium* ou *Chromobacterium violaceum,* pyruvate décarboxylase *d'Acetobacter pasteurianus,* collagénase de *Bacillus sp,* hyaluronidase de *Thermasporomyces composti,* lipase et NADH-oxydase de *Thermus thermophilus,* peroxydase de raifort, *Thermus thermophilus,* uracile phosphoryle transférase de *Saccharolobus shibatae,* ou cytosine désaminase de *Escherichia coli.*

**10.** Procédé selon l'une quelconque des revendications 1 à 9, dans lequel l'anisotropie magnétique de chacune des populations de MNP se trouve dans l'intervalle de 34 mT $\pm$ 50 %, et l'AMF externe est **caractérisé par** un intervalle de fréquence compris entre 50 et 800 kHz et une densité de flux magnétique comprise entre 5 et 70 mT, dans lequel, de préférence, le rapport entre la densité de flux magnétique et l'anisotropie magnétique est supérieur à 0,4, de préférence supérieur à 0,5.

**11.** Procédé selon l'une quelconque des revendications 1 à 10, dans lequel les au moins deux populations sensiblement homogènes et colloïdales de nanoparticules magnétiques (MNP) comprises dans le système de a) présentent un noyau en oxyde de fer et sont choisies dans le groupe constitué de :

i) MNP **caractérisées en ce que** chacune des MNP présente en outre un diamètre de particule moyen compris entre 8-15 nm, sont recouvertes de DMSA, fonctionnalisées avec du NTA et du $Cu^{2+}$, et présentent une anisotropie magnétique de 27 mT ;
ii) MNP **caractérisées en ce que** chacune des MNP présente en outre un diamètre de particule moyen compris entre 18-25 nm, sont recouvertes de PMAO, fonctionnalisées avec du NTA et du $Cu^{2+}$, et présentent une anisotropie magnétique de 34 mT ; ou
iii) MNP **caractérisées en ce que** chacune des MNP présente en outre un diamètre de particule moyen compris entre 25-35 nm, sont recouvertes de PAA, fonctionnalisées avec du NTA et du $Cu^{2+}$, et présentent une anisotropie magnétique de 41 mT ;

et dans lequel les un ou plusieurs AMF appliqués en b) sont choisis dans le groupe constitué de :

i) un intervalle de densité de flux magnétique de 40-50 mT et un intervalle de fréquence de 80-120 kHz,
ii) un intervalle de densité de flux magnétique de 5-20 mT, et un intervalle de fréquence de 300-400 kHz,
iii) un intervalle de densité de flux magnétique de 10-20 mT, et un intervalle de fréquence de 400-500 kHz,
iv) un intervalle de densité de flux magnétique de 50-60 mT, et un intervalle de fréquence de 50-100 kHz,
v) un intervalle de densité de flux magnétique de 50-60 mT, et un intervalle de fréquence de 50-100 kHz, ou
vi) un intervalle de densité de flux magnétique de 20-30 mT, et un intervalle de fréquence de 600-800 kHz.
vii) un intervalle de densité de flux magnétique de 5-20 mT, et un intervalle de fréquence de 600-800 kHz.

**12.** Système comprenant au moins deux populations sensiblement homogènes et colloïdales de nanoparticules magnétiques (MNP), **caractérisé en ce que** chacune des populations de MNP est fonctionnalisée avec au moins une enzyme différente, et **caractérisé en ce que** chacune des populations de MNP présente une anisotropie magnétique différente capable de dissiper l'énergie thermique locale sur la surface de chacune des populations de MNP lors de l'application d'un champ magnétique alternatif (AMF) externe suffisant pour activer chacune des enzymes fonctionnalisées sur lesdites MNP.

**13.** Système selon la revendication 12, dans lequel les au moins deux populations sensiblement homogènes et colloïdales de nanoparticules magnétiques (MNP) comprises dans le système de a) présentent un noyau en oxyde de fer et sont choisies dans le groupe constitué de :

i) MNP **caractérisées en ce que** chacune des MNP présente en outre un diamètre de particule moyen compris entre 8-15 nm, sont recouvertes de DMSA, fonctionnalisées avec du NTA et du $Cu^{2+}$, et présentent une anisotropie magnétique de 27 mT ;

ii) MNP **caractérisées en ce que** chacune des MNP présente en outre un diamètre de particule moyen compris entre 18-25 nm, sont recouvertes de PMAO, fonctionnalisées avec du NTA et du $Cu^{2+}$, et présentent une anisotropie magnétique de 34 mT ; ou

iii) MNP **caractérisées en ce que** chacune des MNP présente en outre un diamètre de particule moyen compris entre 25-35 nm, sont recouvertes de PAA, fonctionnalisées avec du NTA et du $Cu^{2+}$, et présentent une anisotropie magnétique de 41 mT.

14. Utilisation *in vitro* du système selon l'une quelconque des revendications 12 ou 13 pour effectuer le procédé enzymatique selon l'une quelconque des revendications 1 à 11.

**Figure 1**

Figure 2

Figure 3

Figure 4

**Figure 4 (cont.)**

Figure 5

**Figure 6**

**Figure 7**

**Figure 8**

**Figure 8 (cont.)**

**Figure 9**

**Figure 10**

A

B

Figure 11

Figure 11 (cont.)

**Figure 12**

**Figure 12 (cont.)**

**Figure 12 (cont.)**

Figure 13

Figure 14

**Fig 14a**

**Fig 14b**

**Fig 14c**

Figure 15

Figure 15 (cont.)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Magnetic micro-macro biocatalysts applied to industrial bioprocesses. **DEL ARCO JON et al.** BIORESOURCE TECHNOLOGY. ELSEVIER, 16 December 2020, vol. 322
- **MUNOZ-MENENDEZ et al.** *Towards improved magnetic fluid hyperthermia: major-loops to diminish variations in local heating Phys. Chem. Chem. Phys.*, 2017, vol. 19, 14527-14532

- **COSTO, R.** ; **HEINKE, D.** ; **GRÜTTNER, C. et al.** Improving the reliability of the iron concentration quantification for iron oxide nanoparticle suspensions: a two-institutions study.. *Anal Bioanal Chem*, 2019, vol. 411, 1895-1903